# EUROPÄISCHE PATENTSCHRIFT

(11) **EP 3 380 825 B1**
(45) Veröffentlichungstag und Bekanntmachung des Hinweises auf die Patenterteilung: **23.09.2020**
(21) Anmeldenummer: 16819271.4
(22) Anmeldetag: 23.11.2016
(51) Int. Cl.: G01N 21/05, G01N 21/65, G01N 21/77

(54) **VORRICHTUNG UND VERFAHREN ZUM ANALYSIEREN BIOLOGISCHER OBJEKTE MIT RAMAN SPEKTROSKOPIE**
DEVICE AND METHOD FOR ANALYZING BIOLOGICAL OBJECTS WITH RAMAN SPECTROSCOPY
DISPOSITIF ET PROCÉDÉ POUR ANALYSER DES OBJETS BIOLOGIQUES PAR SPECTROSCOPIE RAMAN

(30) Priorität: 23.11.2015 DE 102015223080
(43) Veröffentlichungstag der Anmeldung: 03.10.2018
(73) Patentinhaber: CellTool GmbH, 82347 Bernried (DE)
(72) Erfinder: SCHÜTZE, Karin, 82327 Tutzing (DE); SCHÜTZE, Raimund, 82327 Tutzing (DE)
(74) Vertreter: Ladendorf, Oliver
(86) Internationale Anmeldenummer: PCT/EP2016/078599
(87) Internationale Veröffentlichungsnummer: WO 2017/089427

(56) Entgegenhaltungen:
- WO-A1-2012/033409
- DE-B3-102011 008 788
- US-A1- 2002 030 811
- US-A1- 2005 123 917
- US-A1- 2009 010 388
- US-A1- 2013 171 685
- US-A1- 2014 322 729
- TING-TING LIU ET AL: "A High Speed Detection Platform Based on Surface-Enhanced Raman Scattering for Monitoring Antibiotic-Induced Chemical Changes in Bacteria Cell Wall", PLOS ONE, Bd. 4, Nr. 5, Mai 2009 (2009-05), XP055087067, ISSN: 1932-6203, DOI: 10.1371/journal.pone.0005470
- JANICE L. PANZA ET AL: "Raman spectroscopy and Raman chemical imaging of apoptotic cells", BIOMEDICAL PHOTONICS AND OPTOELECTRONIC IMAGING : 8 - 10 NOVEMBER 2000, BEIJING, CHINA, Bd. 6441, 8. Februar 2007 (2007-02-08), Seite 644108, XP055259734, Bellingham, Wash., US DOI: 10.1117/12.701219 ISBN: 978-1-62841-832-3

## Beschreibung

### GEBIET DER ERFINDUNG

Ausführungsbeispiele der Erfindung betreffen Vorrichtungen und Verfahren zur Überprüfung biologischer Objekte. Ausführungsbeispiele der Erfindung betreffen insbesondere Vorrichtungen und Verfahren, mit denen Zellen, Zellklone, Bakterien oder andere biologische Objekte im Hinblick analysiert werden können, um beispielsweise Reaktionen der Zellen auf Wirkstoffe, Verträglichkeit von Kosmetika und anderen Substanzen, Giftigkeit von chemischen Substanzen oder Erkrankungen wie eine Sepsis oder Wirkstoffresistenzen untersuchen und/oder erkennen zu können. Derartige Vorrichtungen und Verfahren können optional auch eingerichtet sein, um biologische Objekte für eine weitergehende Untersuchung zu sortieren oder um personalisierte Therapien zu ermöglichen.

### HINTERGRUND

Verfahren zum Analysieren biologischer Objekte, beispielsweise Verfahren zum Analysieren von Zellen, Zellklonen, Bakterien oder anderen biologischen Objekten, finden weithin Anwendung. Beispiele für Anwendungen, in denen es wünschenswert ist, biologische Objekte im Hinblick auf ihre Eigenschaften oder ihr Verhalten zu analysieren, beinhalten die Ermittlung von Reaktionen auf Wirkstoffe, Wirkstoffresistenzen, die Erkennung von pathologischen Zuständen, z.B. einer Sepsis, und die Ermittlung von Verträglichkeit von Wirkstoffen und Chemikalien.

Aus Gründen der Reproduzierbarkeit und Zuverlässigkeit kann es wünschenswert sein, Messungen auch an mehreren biologischen Objekten auszuführen und/oder dasselbe biologische Objekt wiederholt einer Messung zu unterziehen. Beispielsweise kann es wünschenswert sein, Reaktionen oder Resistenzen gegenüber mehreren Wirkstoffen zu erkennen, oder Reaktionen über einen längeren Zeitraum zu beobachten. Bei derartigen Messungen an mehreren biologischen Objekten und/oder Wiederholungen von Messungen am selben Objekt können zeitliche Faktoren von besonderer Bedeutung sein. Durch die zeitlich wiederholte Messung kann eine Verlaufsmessung durchgeführt werden, die in der Technik auch als "Monitoring" bezeichnet wird. Zeitliche Messungen an biologischen Objekten sind mit Raman Spektroskopie aus US2005/0123917, US2009/0010388 oder US2013/0171685 bekannt.

### ZUSAMMENFASSUNG

Es besteht ein Bedarf an Vorrichtungen und Verfahren zum Untersuchen von biologischen Objekten. Es besteht dabei insbesondere ein Bedarf an Vorrichtungen und Verfahren, die eine Untersuchung an biologischen Objekten in einer physiologischen Umgebung wie etwa einem Zellkulturmedium erlauben. Diese biologischen Objekte sind besonders in einem nativen bzw. vitalen Zustand von Interesse, welcher über die Untersuchung hinaus beibehalten werden kann. Es besteht insbesondere ein Bedarf an derartigen Vorrichtungen und Verfahren, die für eine Messung an mehreren biologischen Objekten und/oder eine Wiederholung einer Messung am selben biologischen Objekt geeignet sind, wobei anhand quantitativer Messwerte in objektiver Weise Aussagen über Eigenschaften oder das Verhalten der biologischen Objekte gewonnen werden können. Optional wäre eine Eignung zur Sortierung der biologischen Objekte wünschenswert.

Nach Ausführungsbeispielen der Erfindung werden Vorrichtungen und Verfahren bereitgestellt, bei denen biologische Objekte anhand von Raman-Spektren analysiert werden. Eine Arretiereinrichtung ist eingerichtet, um jedes von mehreren biologischen Objekten wenigstens vorübergehend zu arretieren. Der Begriff "arretieren" wie hierin verwendet bezieht sich auf ein zerstörungsfreies Festhalten eines biologischen Objektes. Dabei bleiben metabolische und/oder zelluläre Funktionen und/oder Interaktionen mit der Umgebung des biologischen Objektes erhalten. Weiterhin wird eine Lyse, chemische Fixierung, Zerstörung oder Zertrümmerung der biologischen Objekte vermieden. Durch das "Arretieren" eines biologischen Objektes ist somit eine erfindungsgemäße Analyse biologischer Objekte mittels Raman-Spektroskopie auch über einen längeren Zeitraum hinweg ermöglicht. Arretierte biologische Objekte können zum Beispiel für wenige Minuten, mehrere Stunden, z.B. 4, 5, 6, 8, 10, 12, 15, 20, 24 h, für einen Tag oder 2, 3, 4, 5, 6, oder 7 Tage oder mehr als eine Woche zerstörungsfrei analysiert werden und erlauben dadurch eine Langzeitanalyse oder eine wiederholte Analyse, in bestimmten Ausführungsformen auch mit unterschiedlichen Konzentrationen oder unterschiedlichen Kombinationen von Wirkstoffen wie hierin beschrieben auf biologische Objekte. In speziellen Ausführungsformen kann eine Fixierung der biologischen Objekte durch chemische oder biochemische Interaktion mit einem Haltebereich erfolgen. In diesen Ausführungsformen werden die biologischen Objekte derart festgehalten, dass sie sich in einem feuchten Zustand befinden, der einen Austausch von Molekülen, Subtanzen und/oder Metaboliten der biologischen Objekte, z.B. lebenden Zellen, mit der Umgebung erlaubt. Ebenfalls vorgesehen ist, in speziellen Ausführungsformen, eine anschließende Rückgängigmachung der Fixierung, um Zellen in vitalem und/oder wachstumsfähigem Zustand zu erhalten. Eine erfindungsgemäße Arretiereinrichtung kann dabei als Probenaufnahmeeinrichtung eingerichtet sein, welche es erlaubt, zu untersuchende biologische Objekte, etwa lebende Zellen oder Zellcluster, für einen begrenzten Zeitraum, etwa die Untersuchungsperiode oder eine Abfolge von Untersuchungsperioden, aufzunehmen und in einer Form und an einer Position zu halten, um Wirkstoffexposition und Raman-Spektroskopie-Analyse der biologischen Objekte zu erlauben,

In weiteren Ausführungsformen können sich biologische Objekte frei in Lösung befinden und mittels physikalischen räumlichen Grenzen voneinander separiert werden. Derartige Grenzen können beispielsweise µ-Gefäße oder laminare Ströme oder optisch-induzierte Haltekräfte wie etwa eine Optische Pinzette sein, die es ermöglichen, die biologischen Objekte innerhalb eines bestimmten Raums zu halten. Weiterhin erlauben sie es, die biologischen Objekte nach oder während der Analyse eindeutig zu identifizieren. Damit kann ein derart identifiziertes biologisches Objekt spezifischer weiterer Analyse oder Kultivierung, Aufbewahrung etc. zugeführt werden. Vorteilhafterweise ist in derartigen Ausführungsformen wenigstens ein Teil dieser Reaktionsräume offen, z.B. auf einer Seite oder zwei oder mehr Seiten. Über diesen offenen Bereich bzw. diese offenen Bereiche kann beispielweise ein Fluidstrom fließen, der eine zu testende Substanz enthält, welche über Diffusion in den Reaktionsraum oder die Reaktionsräume eindringt. Der Fluidstrom selbst trägt dabei die biologischen Objekte nicht aus den Reaktionsräumen heraus, da in der Mikrofluidik ein laminares Strömungsprofil herrscht, welches nicht in der Lage ist Objekte außerhalb des Stroms zu befördern. Bevorzugt werden zu testende Substanzen, die mit dem Fluidstrom befördert werden, durch Diffusion zwischen Fluidstrom und Reaktionsraum ausgetauscht. In weiteren Ausführungsformen wird dadurch die Bereitstellung von unterschiedlichen Substanzen, mehreren Substanzen gleichzeitig, unterschiedlichen Konzentrationen von Substanzen, Gradienten von Substanzkonzentrationen, und/oder die zeitlich kontrollierte und/oder periodische Anlieferung von Substanzen wie hierin beschrieben ermöglicht.

Zur Arretierung wird das biologische Objekt jeweils auf einen räumlichen Bereich festgelegt, der eine Veriefung oder ein Mikrowell ist.

In der Erfindung ist ein Laminarstrom in einem Microfluidic-Kanal gegeben, welcher die biologischen Objekte derart anordnet, dass jeweils einzelne Objekte nacheinander zeitlich getrennt fließen bzw. transportiert werden. Die biologischen Objekte können in dieser Ausgestaltung wie Perlen in einer Perlenkette angeordnet sein. Das erfindungsgemäße Raman-Spektroskopiesystem ist somit ausgestaltet und wird dazu verwendet, die entsprechend angeordneten biologischen Objekte einzeln zu erfassen, sie ggf. mittels optischer Haltekräfte zu positionieren, und sie während einer Raman-Messung zu arretieren. Das erlaubt eine simultane Analyse der biologischen Objekte während des Transportprozesses.

Dasselbe biologische Objekt oder unterschiedliche biologische Objekte können wiederholt von einem Raman-Spektroskopiesystem angefahren werden, um mehrere Raman-Spektren, die demselben biologischen Objekt oder unterschiedlichen biologischen Objekten zugeordnet sind, zu erfassen. Zur Automatisierung kann mit einer Bilderfassungseinrichtung ein Übersichtsbild der von einer Arretiereinrichtung arretierten biologischen Objekte erfasst werden. Das Übersichtsbild kann automatisch ausgewertet werden, um Steuersignale für einen Aktor zu erzeugen, der eine Relativbewegung zwischen der Arretiereinrichtung und dem Raman-Spektroskopiesystem herbeiführt. Das Raman-Spektroskopiesystem ist dabei ein System, das eine zerstörungsfreie Analyse von biologischen Objekten erlaubt. Ein derartiges System basiert auf einer Arretierung und Analyse von biologischen Objekten, welche ein Überleben der untersuchten biologischen Objekte erlaubt, d.h. keine Lyse, Zertrümmerung oder chemische Zersetzung oder ähnliche Zerstörung der Aktivität der biologischen Objekte bedingt. In spezifischen Ausführungsformen ist somit eine Weiterverwendung oder Wiedergewinnung der biologischen Objekte ermöglicht. Das erfindungsgemäße Raman-Spektroskopiesystem ist kein SERS-Raman-Spektroskopiesystem.

Die Raman-Spektren können jeweils an den biologischen Objekten selbst und/oder an Material, das die biologischen Objekte umgibt, erfasst werden. Beispielsweise können ein oder mehrere Raman-Spektren an Zellen oder Zellklonen erfasst werden. Alternativ oder zusätzlich können ein oder mehrere Raman-Spektren in einem Fluid, das eine Zelle oder einen Zellklon wenigstens teilweise umgibt, erfasst werden. Auf diese Weise können von einer oder mehreren Zellen ausgeschleuste Metabolite erfasst werden. Die Reaktion biologischer Objekte auf eine oder mehrere Substanzen kann anhand von Metaboliten bestimmt werden, die in einer Umgebung der biologischen Objekte jeweils erfasst werden. Die erfindungsgemäße Vorrichtung sowie erfindungsgemäße Verfahren zum Analysieren biologischer Objekte basieren somit nicht auf einem Nachweis der eingesetzten Substanzen selbst, sondern auf einer Analyse der Reaktion eines biologischen Objektes auf diese Substanzen.

Die Vorrichtungen und Verfahren können eine automatische Sortierung biologischer Objekte abhängig von den an den biologischen Objekten selbst erfassten Raman-Spektren und/oder abhängig von den Raman-Spektren, die an einem die biologischen Objekte umgebenden Fluid erfasst werden, ausführen. Zum Sortieren biologischer Objekte können Fluidströme in einem Fluidikchip, optische Strahlung, beispielsweise Optische Pinzetten, elektrische oder magnetische Felder oder andere Techniken eingesetzt werden. Das Sortieren biologischer Objekte gemäß der vorliegenden Erfindung, welches auch automatisiert durchgeführt werden kann, kann somit in Abhängigkeit von den gemessenen Raman-Spektren erfolgen und ermöglicht es sowohl Zellen aufgrund Ihrer Reaktion auf eine Substanz selektiv zu entnehmen und/oder zu sortieren, als auch Zellen selektiv zu entnehmen und/oder zu sortieren, welche abweichende, auffällige, bereits zuvor als interessant definierte etc. Raman-Spektren aufweisen.

Die Vorrichtungen und Verfahren nach Ausführungsbeispielen können für eine Vielzahl von Anwendungen eingesetzt werden. Beispielhaft können die Vorrichtungen und Verfahren verwendet werden, um Wirkstoffresistenzen zu erkennen, um wirksame personalisierte Therapien zu identifizieren, um die Verträglichkeit chemischer Stoffe zu bewerten und/oder um eine Sepsis oder andere pathologische Zustände automatisch durch eine Erfassung und Auswertung von Raman-Spektren zu erkennen.

Die Vorrichtungen und Verfahren gemäß der vorliegenden Erfindung ermöglichen damit eine Arretierung und Analyse von biologischen Objekten im dreidimensionalen Raum. Dieser Aspekt, der in bestimmten Ausführungsformen umgesetzt ist, erlaubt es zum Beispiel Raman-Spektren an Zellen oder Zellklonen zu erfassen, die in einer Zellschicht oder mehr als einer Zellschicht vorliegen. So können Raman-Spektren, die einen akkuraten Aufschluss über Wirkstoffreaktionen oder -resistenzen von biologischen Objekten liefern, in dreidimensionalen Räumen von 1, 2, 3, 4, 5 oder mehr Zellschichten erhalten werden. Derartige Zellschichten können beispielsweise zwischen 1 µm und 500 µm Dicke aufweisen. In besonderen Ausführungsformen können Zellschichten einer Dicke von 1 µm, 2 µm, 3 µm, 4 µm, 5 µm, 10 µm, 15 µm, 20 µm, 30 µm, 40 µm, 50µm, 100µm, 150 µm, 200µm, 250µm, 300µm. 350 µm, 400µm, 450 µm oder 500 µm vorliegen. Die Dicke der Zellschichten ist dabei im Regelfall abhängig vom Zelltyp und dem zu untersuchenden Probentypus. So können im Fall von Einzelzellen Dicken bzw. Durchmesser im Bereich von 1-15 µm vorliegen und analysiert werden. Im Fall von Zellclustern, insbesondere kleinen Zellclustern, oder Spheroiden, welche vom vorliegend beschriebenen System ebenfalls analysiert werden können, kann eine Dicke bzw. ein Durchmesser der Schicht im Bereich von 50 - 300 µm vorliegen. Ebenfalls vorgesehen ist die Analyse von Zellschichten die eine Dicke aufweisen, welche zwischen den oben genannten Werten liegt, bzw. kleiner oder größer ist. Damit ist ebenfalls eine *in situ*-Analyse von mehrschichtigen Akkumulationen biologischer Objekte möglich.
Eine Vorrichtung zum Analysieren biologischer Objekte nach einem Ausführungsbeispiel umfasst ein Raman-Spektroskopiesystem zum Erfassen wenigstens eines Raman-Spektrums. Die Vorrichtung umfasst eine Arretiereinrichtung, die eingerichtet ist, um die biologischen Objekte wenigstens vorübergehend zu arretieren. Die Vorrichtung umfasst eine elektronische Recheneinrichtung, die eingerichtet ist, um abhängig von einer Auswertung des wenigstens einen Raman-Spektrums eine Reaktion eines von der Arretiereinrichtung arretierten biologischen Objekts auf wenigstens eine Substanz zu ermitteln.

Die Vorrichtung kann eingerichtet sein, um ein Raman-Spektrum wahlweise an einem biologischen Objekt oder an einem das biologische Objekt überdeckenden oder umgebenden Fluid zu messen. Das Fluid kann eine Flüssigkeit sein.

Die Vorrichtung kann eingerichtet sein, um ein Raman-Spektrum vor Gabe der wenigstens einen Substanz und ein weiteres Raman-Spektrum nach Gabe der wenigstens einen Substanz zu erfassen. Die Recheneinrichtung kann eingerichtet sein, um durch einen Vergleich des vor Gabe der wenigstens einen Substanz erfassten Raman-Spektrums und des nach Gabe der wenigstens einen Substanz erfassten Raman-Spektrums die Reaktion des biologischen Objekts auf die wenigstens eine Substanz zu ermitteln.

Die Vorrichtung kann eingerichtet sein, um ein einem biologischen Objekt zugeordnetes Raman-Spektrum unter Verwendung eines weiteren Raman-Spektrums, das eine Raman-Streuung an der Arretiereinrichtung repräsentiert, zu verarbeiten. Dazu kann ein Aktor so angesteuert werden, dass das weitere Raman-Spektrum an einer Stelle der Arretiereinrichtung, die kein biologisches Objekt hält, erfasst wird. Die Recheneinrichtung kann für alle Raman-Spektren, die biologischen Objekten zugeordnet sind, jeweils eine Hintergrundkorrektur ausführen, die von dem weiteren Raman-Spektrum, das die Raman-Streuung an der Arretiereinrichtung repräsentiert, abhängt. Beispielsweise kann ein Differenzspektrum ermittelt werden, um das Raman-Spektrum eines biologischen Objekts oder eines Metaboliten und die Raman-Streuung an der Arretiereinrichtung zu trennen.

Die Recheneinrichtung kann eingerichtet sein, um die Reaktion anhand einer statistischen Auswertung des vor Gabe des wenigstens einen Substanz erfassten Raman-Spektrums und des nach Gabe der wenigstens einen Substanz erfassten Raman-Spektrums die Reaktion des biologischen Objekts auf die wenigstens eine Substanz zu ermitteln. Die statistische Auswertung kann eine Hauptkomponentenanalyse, eine Clusteranalyse und/oder eine lineare Diskriminanzanalyse (LDA-Analyse) umfassen.

Die Vorrichtung kann eingerichtet sein, um das von der Arretiereinrichtung arretierte biologische Objekt wiederholt zum Erfassen des wenigstens einen Raman-Spektrums anzufahren. Die Vorrichtung kann eingerichtet sein, um ein- und dasselbe biologische Objekt zur Erfassung eines Raman-Spektrums vor Gabe der wenigstens einen Substanz und ein weiteren Raman-Spektrums nach Gabe der wenigstens einen Substanz anzufahren, wobei ein Aktor zwischen den beiden Erfassungen eine Relativbewegung zwischen der Arretiereinrichtung und dem Raman-Spektroskopiesystem herbeiruft.

Die Vorrichtung kann einen von der elektronischen Recheneinrichtung steuerbare Aktor umfassen, der zum Hervorrufen einer Relativbewegung zwischen der Arretiereinrichtung und dem Raman-Spektroskopiesystem eingerichtet ist. Der Aktor kann so angesteuert werden, dass die Zeit, in der ein biologisches Objekt auf eine Substanz reagiert, bevor ein weiteres Raman-Spektrum erfasst wird, zur Erfassung von Raman-Spektren an anderen biologischen Objekten genutzt wird. Dazu kann der Aktor Erfassungen einer Relativbewegung zwischen der Arretiereinrichtung und dem Raman-Spektroskopiesystem herbeirufen, um Messzeiten effektiv zu nutzen.

Die elektronische Recheneinrichtung kann eingerichtet sein, um den Aktor abhängig von Abständen zwischen wenigstens zwei durch die Arretiereinrichtung arretierten biologischen Objekten zu steuern.

Die elektronische Recheneinrichtung kann eingerichtet sein, um den Aktor so zu steuern, dass ein einem ersten biologischen Objekt zugeordnetes Raman-Spektrum erfasst wird, während gleichzeitig ein zweites biologisches Objekt, das an der Arretiereinrichtung beabstandet von dem ersten biologischen Objekt gehalten wird, der wenigstens einen Substanz ausgesetzt wird.

Die Vorrichtung kann eine mit der elektronischen Recheneinrichtung gekoppelte Bilderfassungseinrichtung umfassen, die eingerichtet ist, um ein Bild der durch die Arretiereinrichtung arretierten biologischen Objekte zu erfassen. Die elektronische Recheneinrichtung kann eingerichtet sein, um den Aktor abhängig von dem erfassten Bild zu steuern.

Die elektronische Recheneinrichtung kann eingerichtet sein, um den Aktor so zu steuern, dass sequentiell Raman-Spektren erfasst werden, die unterschiedlichen biologischen Objekten zugeordnet sind. In mehreren aufeinanderfolgenden Erfassungssequenzen kann jedes von mehreren biologischen Objekten jeweils mehrfach zur Erfassung von Raman-Spektren angefahren werden, um die Reaktion auf eine oder mehrere Substanzen im Zeitverlauf, d.h. zeitabhängig zu ermitteln.

Die Bilderfassungseinrichtung kann einen von einem Objektiv des Raman-Spektroskopiesystems separaten optischen Strahlengang aufweisen. Auf diese Weise kann eine rasche Navigation anhand des von der Bilderfassungseinrichtung erfassten Übersichtsbilds kombiniert werden mit einer hohen örtlichen Auflösung durch das Raman-Spektroskopiesystem.

Die Bilderfassungseinrichtung kann mehrere Linsen aufweisen, die alle von dem Objektiv des Raman-Spektroskopiesystems verschieden sind.

Die Vorrichtung kann so ausgestaltet sein, dass wenigstens eine Linse der Optik der Bilderfassungseinrichtung auch zum Einstrahlen von Anregungslicht und/oder zum Erfassen von Streulicht bei der Raman-Spektroskopie verwendet wird.

Die Arretiereinrichtung umfasst Haltebereiche, an denen jeweils mindestens ein biologisches Objekt arretierbar ist.

Die Vorrichtung kann eingerichtet ist, um einen die Mehrzahl von Haltebereichen überströmenden, umströmenden oder durchströmenden Fluidstrom zu erzeugen. Die Vorrichtung kann eine Pumpe oder eine andere Einrichtung zum Hervorrufen eines Fluidstroms umfassen, der die Mehrzahl von Haltebereichen überströmt.

Die Vorrichtung kann eingerichtet sein, um ein biologisches Objekt aus dem Fluidstrom zu einem Haltebereich und/oder von dem Haltebereich in den Fluidstrom zu bewegen.

Die Vorrichtung kann eingerichtet sein, um das biologische Objekt in einer Richtung quer zu einer Strömungsrichtung des Fluidstroms zu bewegen, um das biologische Objekt wenigstens vorübergehend zu arretieren und/oder um das biologische Objekt von der Arretiereinrichtung zu lösen. Die Vorrichtung kann eingerichtet sein, um das biologische Objekt in einer Richtung senkrecht zu einer Strömungsrichtung des Fluidstroms zu bewegen, um das biologische Objekt wenigstens vorübergehend zu arretieren und/oder um das biologische Objekt von der Arretiereinrichtung zu lösen.

Die Vorrichtung kann eingerichtet sein, um ein biologisches Objekt zerstörungsfrei reversibel aus dem Fluidstrom zu einem Haltebereich der Arretiereinrichtung und wieder zurück in den Fluidstrom zu bewegen.

Die Vorrichtung kann eine Quelle elektromagnetischer Strahlung umfassen, die eingerichtet ist, um ein biologisches Objekt aus dem Fluidstrom zu einem Haltebereich und/oder von dem Haltebereich in den Fluidstrom zu bewegen.

Die Quelle elektromagnetischer Strahlung kann eine Optische Pinzette umfassen.

Die Haltebereiche können jeweils so dimensioniert sind, dass an jedem Haltebereich nur genau ein biologisches Objekt arretierbar ist.

Die Arretiereinrichtung kann eine Mehrzahl von Vertiefungen umfassen, die jeweils als Haltebereich zum vorübergehenden Arretieren eines biologischen Objekts dienen.

Die Mehrzahl von Vertiefungen kann jeweils so dimensioniert sein, dass jeweils nur genau eine Zelle oder genau ein Bakterium in der entsprechenden Vertiefung aufnehmbar ist.

Die Mehrzahl von Vertiefungen kann so dimensioniert sein, dass mehrere biologische Objekte, beispielsweise ein Zellklon mit mehreren Zellen, darin aufnehmbar ist. Die Abmessungen der Mehrzahl von Vertiefungen können so gewählt sein, dass sie die Größe eines darin aufgenommenen Zellklons begrenzen.

Die Haltebereiche können jeweils so dimensioniert sein, dass an jedem Haltebereich nur genau eine Zelle arretierbar ist.

Die Haltebereiche können jeweils so dimensioniert sein, dass an jedem der Haltebereiche mehrere biologische Objekte arretierbar sind.

Die Haltebereiche können jeweils so dimensioniert sein, dass an jedem der Haltebereiche mehrere Zellen arretierbar sind.

Die Arretiereinrichtung kann eine Microslide umfassen.

Die Arretiereinrichtung kann eine Microwellplatte umfassen.

Die Vorrichtung umfasst eine Zuführeinrichtung zum Zuführen der wenigstens einen Substanz zu den biologischen Objekten.

Die Zuführeinrichtung kann eingerichtet sein, um mehrere unterschiedliche Substanzen zu den biologischen Objekten zuzuführen.

Die Zuführeinrichtung kann wenigstens ein Reservoir für eine Substanz umfassen, um die Substanz einem biologischen Objekt zuzuführen.

Die Zuführeinrichtung kann so gestaltet sein, dass eine Anreicherung von vereinzelt in einer großen Probenmenge vorliegenden Zellen ermöglicht wird. Dies kann beispielsweise durch Elimination nicht interessanter Zellen in einem vorgeschalteten Lyseschritt oder durch Nutzen von Zentrifugalkräften oder Zentripetalkräften durch entsprechende Konfiguration von Fluidik-Kanälen geschehen. Alternativ oder zusätzlich kann eine derartige Anreicherung unter Verwendung osmotischer Kräfte mittels selektiver Membrandiffusion vonstatten gehen.

Die Zuführeinrichtung kann Reservoire für mehrere unterschiedliche Substanzen umfassen. Die Zuführeinrichtung kann eingerichtet sein, um einem ersten biologischen Objekt eine erste Substanz zuzuführen und um einem von dem ersten biologischen Objekt beabstandet an der Arretiereinrichtung gehaltenen zweiten biologischen Objekt eine zweite Substanz zuzuführen, die von der ersten Substanz verschieden ist. Die Zuführeinrichtung kann eingerichtet sein, um die unterschiedlichen Substanzen zeitlich koordiniert, beispielsweise gleichzeitig oder zeitversetzt, unterschiedlichen biologischen Objekten zuzuführen.

Wenigstens eine Substanz kann ein Wirkstoff sein. Die Substanzen können mehrere voneinander verschiedene Wirkstoffe umfassen. Wirkstoffe können beispielsweise Biomoleküle, biochemische Stoffe, Proteine, Aminosäuren oder ähnliches sein.

Alternativ oder zusätzlich kann wenigstens eine Substanz eine Chemikalie sein, deren Verträglichkeit getestet werden soll. Die Substanzen können mehrere voneinander verschiedene Chemikalien umfassen.

Alternativ oder zusätzlich kann wenigstens eine Substanz ein Toxin sein, dessen Wirkung getestet werden soll. Die Substanzen können mehrere voneinander verschiedene Toxine umfassen. Ein Toxin kann eine Chemikalie, zum Beispiel ein kleines organisches oder anorganisches Molekül sein. Ein Toxin kann ebenfalls ein biologisches Molekül, z.B. ein Peptid oder Protein sein. Weiterhin kann die Substanz ein biologischer Wirkstoff, zum Beispiel ein lebender Organismus, eine Zelle, ein Bakterium, ein Virus, ein Bakteriophage, oder Teile davon, oder Kombinationen davon sein. Beispiele von Bakterien umfassen Gram-positive Staphylokokken, Streptokocken und Enterokokken sowie Gram-negative Escherichia, Klebsiella, Serratia und Pseudomonas.

Die elektronische Recheneinrichtung kann eingerichtet sein, um abhängig von der Auswertung des wenigstens einen Raman-Spektrums die Reaktion auf mehrere Substanzen zu ermitteln und/oder um abhängig von der Auswertung mehrerer Raman-Spektren einen Verlauf der Reaktion zeitabhängig zu verfolgen.

Für eine Mehrzahl biologischer Objekte kann die Recheneinrichtung jeweils ein erstes Raman-Spektrum vor Gabe einer Substanz und ein zweites Raman-Spektrum nach Gabe einer Substanz durch eine statistische Auswertung vergleichen. Die von der Recheneinrichtung ausgeführte statistische Auswertung kann eine Hauptkomponentenanalyse, eine Clusteranalyse und/oder eine lineare Diskriminanzanalyse (LDA-Analyse) umfassen.

Die elektronische Recheneinrichtung kann eingerichtet sein, um abhängig von der Auswertung des wenigstens einen Raman-Spektrums eine Wirkstoffresistenz zu erkennen.

Die elektronische Recheneinrichtung kann eingerichtet sein, um abhängig von der Auswertung mehrerer Raman-Spektren zu erkennen, gegenüber welchen von mehreren Wirkstoffen eine Resistenz vorliegt.

Die elektronische Recheneinrichtung kann eingerichtet sein, um abhängig von der Auswertung mehrerer Raman-Spektren zu ermitteln, welche Chemikalien für einen Organismus unverträglich sind.

Die elektronische Recheneinrichtung kann eingerichtet sein, um abhängig von der Auswertung mehrerer Raman-Spektren zu ermitteln, welche Wirkung Toxine für einen Organismus aufweisen.

Die elektronische Recheneinrichtung kann eingerichtet sein, um abhängig von der Auswertung mehrerer Raman-Spektren zu ermitteln, welche Wirkung ein biologischer Wirkstoff für einen Organismus besitzt.

Die elektronische Recheneinrichtung kann eingerichtet sein, um zum Erkennen der Wirkstoffresistenz ein vor Zuführen der wenigstens einen Substanz erfasstes erstes Raman-Spektrum mit einem nach einer Wirkstoffgabe erfassten zweiten Raman-Spektrum zu vergleichen.

Die wenigstens eine Substanz kann einen Wirkstoff, ein Toxin, einen biologischen Wirkstoff und/oder eine Chemikalie umfassen.

Die Vorrichtung kann derart eingerichtet sein, dass das wenigstens eine durch Raman-Streuung an einem biologischen Objekt erfasste Raman-Spektrum von der elektronischen Recheneinrichtung ausgewertet wird.

Die Vorrichtung kann derart eingerichtet sein, dass das wenigstens eine von der elektronischen Recheneinrichtung ausgewertete Raman-Spektrum durch Raman-Streuung an einem von dem biologischen Objekt verschiedenen Material erfasst wird.

Die Vorrichtung kann eingerichtet sein, um parallel die Reaktion mehrerer biologischer Objekte auf eine Substanz oder mehrere Substanzen zu ermitteln.

Ein Verfahren zum Analysieren biologischer Objekte umfasst ein Arretieren eines biologischen Objekts durch eine Arretiereinrichtung. Das Verfahren umfasst ein Erfassen wenigstens eines Raman-Spektrums des arretierten biologischen Objekts. Das Verfahren umfasst ein Auswerten des wenigstens einen Raman-Spektrums zum Ermitteln einer Reaktion eines biologischen Objekts auf wenigstens eine Substanz.

Das Verfahren kann mit der Vorrichtung nach einem Ausführungsbeispiel ausgeführt werden.

Bei dem Verfahren kann ein Raman-Spektrum wahlweise an einem biologischen Objekt oder an einem das biologische Objekt überdeckenden oder umgebenden Fluid erfasst werden. Das Fluid kann eine Flüssigkeit sein.

Bei dem Verfahren kann ein Raman-Spektrum vor Gabe der wenigstens einen Substanz und ein weiteres Raman-Spektrum nach Gabe der wenigstens einen Substanz erfasst werden. Eine Recheneinrichtung kann durch einen Vergleich des vor Gabe des wenigstens einen Substanz erfassten Raman-Spektrums und des nach Gabe der wenigstens einen Substanz erfassten Raman-Spektrums die Reaktion des biologischen Objekts auf die wenigstens eine Substanz ermitteln.

Bei dem Verfahren kann ein einem biologischen Objekt zugeordnetes Raman-Spektrum unter Verwendung eines weiteren Raman-Spektrums, das eine Raman-Streuung an der Arretiereinrichtung repräsentiert, verarbeitet werden. Dazu kann ein Aktor so angesteuert werden, dass das weitere Raman-Spektrum an einer Stelle der Arretiereinrichtung, die kein biologisches Objekt hält, erfasst wird. Eine Recheneinrichtung kann für alle Raman-Spektren, die biologischen Objekten zugeordnet sind, jeweils eine Hintergrundkorrektur ausführen, die von dem weiteren Raman-Spektrum, das die Raman-Streuung an der Arretiereinrichtung repräsentiert, abhängt. Beispielsweise kann ein Differenzspektrum ermittelt werden, um das Raman-Spektrum eines biologischen Objekts oder eines Metaboliten und die Raman-Streuung an der Arretiereinrichtung zu trennen.

Bei dem Verfahren kann die Reaktion auf die Substanz(en) anhand einer statistischen Auswertung des vor Gabe des wenigstens einen Substanz erfassten Raman-Spektrums und des nach Gabe der wenigstens einen Substanz erfassten Raman-Spektrums die Reaktion des biologischen Objekts auf die wenigstens eine Substanz zu ermitteln. Die statistische Auswertung kann eine Hauptkomponentenanalyse, eine Clusteranalyse und/oder eine lineare Diskriminanzanalyse (LDA-Analyse) umfassen.

Bei dem Verfahren kann das von der Arretiereinrichtung arretierte biologische Objekt wiederholt zum Erfassen des wenigstens einen Raman-Spektrums angefahren werden. Bei dem Verfahren kann ein- und dasselbe biologische Objekt zur Erfassung eines Raman-Spektrums vor Gabe der wenigstens einen Substanz und eines weiteren Raman-Spektrums nach Gabe der wenigstens einen Substanz angefahren werden, wobei ein Aktor zwischen den beiden Erfassungen eine Relativbewegung zwischen der Arretiereinrichtung und dem Raman-Spektroskopiesystem herbeiruft.

Bei dem Verfahren kann ein steuerbarer Aktor zum Hervorrufen einer Relativbewegung zwischen der Arretiereinrichtung und dem Raman-Spektroskopiesystem gesteuert werden. Der Aktor kann so angesteuert werden, dass die Zeit, in der ein biologisches Objekt auf eine Substanz reagiert, bevor ein weiteres Raman-Spektrum erfasst wird, zur Erfassung von Raman-Spektren an anderen biologischen Objekten genutzt wird. Dazu kann der Aktor Erfassungen eine Relativbewegung zwischen der Arretiereinrichtung und dem Raman-Spektroskopiesystem herbeirufen, um Messzeiten effektiv zu nutzen.

Bei dem Verfahren kann der Aktor abhängig von Abständen zwischen wenigstens zwei durch die Arretiereinrichtung arretierten biologischen Objekten gesteuert werden.

Bei dem Verfahren kann der Aktor so gesteuert werden, dass ein einem ersten biologischen Objekt zugeordnetes Raman-Spektrum erfasst wird, während gleichzeitig ein zweites biologisches Objekt, das an der Arretiereinrichtung beabstandet von dem ersten biologischen Objekt gehalten wird, der wenigstens einen Substanz ausgesetzt wird.

Bei dem Verfahren kann mit einer Bilderfassungseinrichtung ein Bild der durch die Arretiereinrichtung arretierten biologischen Objekte erfasst werden. Der Aktor kann abhängig von dem erfassten Bild gesteuert werden.

Bei dem Verfahren kann der Aktor so gesteuert werden, dass sequentiell Raman-Spektren erfasst werden, die unterschiedlichen biologischen Objekten zugeordnet sind. In mehreren aufeinanderfolgenden Erfassungssequenzen kann jedes von mehreren biologischen Objekten jeweils mehrfach zur Erfassung von Raman-Spektren angefahren werden, um die Reaktion auf eine oder mehrere Substanzen im zeitlichen Verlauf zu ermitteln.

Bei dem Verfahren kann die Bilderfassungseinrichtung einen von einem Objektiv des Raman-Spektroskopiesystems separaten optischen Strahlengang aufweisen. Auf diese Weise kann eine rasche Navigation anhand des von der Bilderfassungseinrichtung erfassten Übersichtsbilds kombiniert werden mit einer hohen örtlichen Auflösung durch das Raman-Spektroskopiesystem.

Die Bilderfassungseinrichtung kann mehrere Linsen aufweisen, die alle von dem Objektiv des Raman-Spektroskopiesystems verschieden sind.

Wenigstens eine Linse der Optik der Bilderfassungseinrichtung kann auch zum Einstrahlen von Anregungslicht und/oder zum Erfassen von Streulicht bei der Raman-Spektroskopie verwendet werden. Die Linse der Optik kann ein Objektiv eines Mikroskops sein, durch das der Anregungsstrahl hindurchgeführt wird.

Bei dem Verfahren umfasst die Arretiereinrichtung Haltebereiche, an denen jeweils mindestens ein biologisches Objekt arretierbar ist.

Das Verfahren kann ein Erzeugen eines Fluidstroms umfassen, der die Mehrzahl von Haltebereichen überströmt, umströmt oder durchströmt. Der Fluidstrom, der die Mehrzahl von Haltebereichen überströmt, kann mit einer Pumpe oder einer anderen Einrichtung zum Hervorrufen eines Fluidstroms erzeugt werden.

Bei dem Verfahren kann ein biologisches Objekt aus dem Fluidstrom zu einem Haltebereich und/oder von dem Haltebereich in den Fluidstrom bewegt werden.

Bei dem Verfahren kann das biologische Objekt in einer Richtung quer zu einer Strömungsrichtung des Fluidstroms bewegt werden, um das biologische Objekt wenigstens vorübergehend zu arretieren und/oder um das biologische Objekt von der Arretiereinrichtung zu lösen. Bei dem Verfahren kann das biologische Objekt in einer Richtung senkrecht zu einer Strömungsrichtung des Fluidstroms bewegt werden, um das biologische Objekt wenigstens vorübergehend zu arretieren und/oder um das biologische Objekt von der Arretiereinrichtung zu lösen.

Bei dem Verfahren kann ein biologisches Objekt zerstörungsfrei reversibel aus dem Fluidstrom zu einem Haltebereich der Arretiereinrichtung und wieder zurück in den Fluidstrom bewegt werden.

Bei dem Verfahren kann eine Quelle elektromagnetischer Strahlung gesteuert werden, um ein biologisches Objekt aus dem Fluidstrom zu einem Haltebereich und/oder von dem Haltebereich in den Fluidstrom zu bewegen.

Die Quelle elektromagnetischer Strahlung kann eine Optische Pinzette umfassen. Bei dem Verfahren können die Haltebereiche jeweils so dimensioniert sein, dass an jedem Haltebereich nur genau ein biologisches Objekt arretierbar ist.

Bei dem Verfahren umfasst die Arretiereinrichtung eine Mehrzahl von Vertiefungen, die jeweils als Haltebereich zum vorübergehenden Arretieren eines biologischen Objekts dienen.

Bei dem Verfahren kann die Mehrzahl von Vertiefungen jeweils so dimensioniert sein, dass jeweils nur genau eine Zelle oder genau ein Bakterium in der entsprechenden Vertiefung aufnehmbar ist.

Bei dem Verfahren kann die Mehrzahl von Vertiefungen so dimensioniert sein, dass mehrere biologische Objekte, beispielsweise ein Zellklon mit mehreren Zellen, darin aufnehmbar ist. Die Abmessungen der Mehrzahl von Vertiefungen können so gewählt sein, dass sie die Größe eines darin aufgenommenen Zellklons begrenzen. Bei dem Verfahren können die Haltebereiche jeweils so dimensioniert sein, dass an jedem Haltebereich nur genau eine Zelle arretierbar ist.

Bei dem Verfahren können die Haltebereiche jeweils so dimensioniert sein, dass an jedem der Haltebereiche mehrere biologische Objekte arretierbar sind.

Bei dem Verfahren können die Haltebereiche jeweils so dimensioniert sein, dass an jedem der Haltebereiche mehrere Zellen arretierbar sind.

Bei dem Verfahren kann die Arretiereinrichtung ein Microslide umfassen.

Bei dem Verfahren kann die Arretiereinrichtung eine Microwellplatte umfassen.

Bei dem Verfahren wird eine Zuführeinrichtung zum Zuführen der wenigstens einen Substanz zu den biologischen Objekten automatisch gesteuert.

Bei dem Verfahren kann die Zuführeinrichtung eingerichtet sein, um mehrere unterschiedliche Substanzen zu den biologischen Objekten zuzuführen.

Bei dem Verfahren kann die Zuführeinrichtung wenigstens ein Reservoir für eine Substanz umfassen, um die Substanz einem biologischen Objekt zuzuführen.

Bei dem Verfahren kann die Zuführeinrichtung Reservoire für mehrere unterschiedliche Substanzen umfassen. Die Zuführeinrichtung kann eingerichtet sein, um einem ersten biologischen Objekt eine erste Substanz zuzuführen und um einem von dem ersten biologischen Objekt beabstandet an der Arretiereinrichtung gehaltenen zweiten biologischen Objekt eine zweite Substanz zuzuführen, die von der ersten Substanz verschieden ist.

Bei dem Verfahren kann die Zuführeinrichtung eingerichtet sein, um die unterschiedlichen Substanzen zeitlich koordiniert, beispielsweise gleichzeitig oder zeitversetzt, unterschiedlichen biologischen Objekten zuzuführen.

Wenigstens eine Substanz kann ein Wirkstoff sein. Die Substanzen können mehrere voneinander verschiedene Wirkstoffe umfassen.

Alternativ oder zusätzlich kann wenigstens eine Substanz ein Stimulanz oder ein Differenzierungsagens sein, das die Zellen zu einer Veränderung stimuliert. Beispielsweise kann wenigstens eine Substanz ein Stimulanz oder ein Differenzierungsagens sein, das die Zellen zu Verhaltensänderung stimuliert. Die Substanzen können mehrere voneinander verschiedene Wirkstoffe umfassen.

Alternativ oder zusätzlich kann wenigstens eine Substanz eine Chemikalie sein, deren Verträglichkeit getestet werden soll. Die Substanzen können mehrere voneinander verschiedene Chemikalien umfassen.

Alternativ oder zusätzlich kann wenigstens eine Substanz ein Toxin sein, dessen Wirkung getestet werden soll. Die Substanzen können mehrere voneinander verschiedene Toxine umfassen. Ein Toxin kann eine Chemikalie, zum Beispiel ein kleines organisches oder anorganisches Molekül sein. Ein Toxin kann ebenfalls ein biologisches Molekül, z.B. ein Peptid oder Protein sein. Weiterhin kann die Substanz ein biologischer Wirkstoff, zum Beispiel ein lebender Organismus, eine Zelle, ein Bakterium, ein Virus, ein Bacteriophage, oder Teile davon, oder Kombinationen davon sein. Beispiele von Bakterien umfassen Gram-positive Staphylokokken, Streptokocken und Enterokokken sowie Gram-negative Escherichia, Klebsiella, Serratia und Pseudomonas.

Bei dem Verfahren können ein oder mehrere Reservoire vorgesehen sein, in die ausgewählte aussortiert Zellen transportiert und abgelegt werden können. Aus diesem Reservoir oder diesen Reservoiren können die ausgewählten Zellen für weitere Untersuchungen entnommen werden.

Die elektronische Recheneinrichtung kann eingerichtet sein, um abhängig von der Auswertung des wenigstens einen Raman-Spektrums die Reaktion auf mehrere Substanzen zu ermitteln und/oder um abhängig von der Auswertung mehrerer Raman-Spektren einen Verlauf der Reaktion zeitabhängig zu verfolgen.

Für eine Mehrzahl biologischer Objekte kann bei dem Verfahren jeweils ein erstes Raman-Spektrum vor Gabe einer Substanz und ein zweites Raman-Spektrum nach Gabe einer Substanz durch eine statistische Auswertung verglichen werden. Eine von einer Recheneinrichtung ausgeführte statistische Auswertung kann eine Hauptkomponentenanalyse, eine Clusteranalyse und/oder eine lineare Diskriminanzanalyse (LDA-Analyse) umfassen.

Bei dem Verfahren kann die elektronische Recheneinrichtung abhängig von der Auswertung des wenigstens einen Raman-Spektrums eine Wirkstoffreaktion erkennen.

Bei dem Verfahren kann die elektronische Recheneinrichtung abhängig von der Auswertung des wenigstens einen Raman-Spektrums eine Wirkstoffresistenz erkennen.

Bei dem Verfahren kann die elektronische Recheneinrichtung abhängig von der Auswertung mehrerer Raman-Spektren erkennen, gegenüber welchen von mehreren Wirkstoffen eine Resistenz vorliegt.

Bei dem Verfahren kann die elektronische Recheneinrichtung abhängig von der Auswertung mehrerer Raman-Spektren ermitteln, welche Chemikalien für einen Organismus unverträglich sind.

Bei dem Verfahren kann die elektronische Recheneinrichtung abhängig von der Auswertung mehrerer Raman-Spektren ermitteln, welche Wirkung ein Toxin oder mehrere Toxine für einen Organismus aufweisen.

Bei dem Verfahren kann die elektronische Recheneinrichtung abhängig von der Auswertung mehrerer Raman-Spektren ermitteln, welche Wirkung ein biologischer Wirkstoff oder mehrere Wirkstoffe für einen Organismus aufweisen.

Bei dem Verfahren kann die elektronische Recheneinrichtung zum Erkennen der Wirkstoffresistenz ein vor Zuführen der wenigstens einen Substanz erfasstes erstes Raman-Spektrum mit einem nach einer Wirkstoffgabe erfassten zweiten Raman-Spektrum vergleichen.

Bei dem Verfahren kann die wenigstens eine Substanz kann einen Wirkstoff, einen biologischen Wirkstoff, ein Toxin und/oder eine Chemikalie umfassen.

Bei dem Verfahren kann das wenigstens eine von der elektronischen Recheneinrichtung ausgewertete Raman-Spektrum durch Raman-Streuung an einem biologischen Objekt erfasst werden.

Bei dem Verfahren kann das wenigstens eine von der elektronischen Recheneinrichtung ausgewertete Raman-Spektrum durch Raman-Streuung an einem von dem biologischen Objekt verschiedenen Material erfasst werden, beispielsweise durch Raman-Streuung an einem Fluid, das das biologische Objekt überdeckt.

Bei dem Verfahren kann parallel die Reaktion mehrerer biologischer Objekte auf eine Substanz oder mehrere Substanzen ermittelt werden.

Mit den Vorrichtungen und Verfahren nach Ausführungsbeispielen können die Reaktionen biologischer Objekte auf eine oder mehrere unterschiedliche Substanzen jeweils zeitabhängig verfolgt werden. Insbesondere können biologische Objekte über einen längeren Zeitraum von mehreren Stunden, Tagen oder Wochen zerstörungsfrei analysiert werden, z.B. in Form einer periodisch wiederholten Analyse, einer Analyse nach mehrfacher Gabe von Substanzen, eskalierender Gabe von Substanzen, der Gabe von unterschiedlichen Substanzen in zeitlicher Abfolge etc. Information über den zeitabhängigen Verlauf und die Kinetik der Reaktion der biologischen Objekte auf die Substanzen kann durch Auswertung mehrerer Raman-Spektren gewonnen werden.

Vorrichtungen und Verfahren nach Ausführungsbeispielen erlauben eine schnelle und markerfreie Überprüfung biologischer Objekte im Hinblick auf ihre Reaktion auf Substanzen wie Wirkstoffe, Toxine oder Chemikalien. Derartige Analyseverfahren können beispielsweise zur Erkennung von Wirkstoffresistenzen, zur Bestimmung chemischer Verträglichkeit, zur automatischer Identifizierung wirksamer personalisierter Therapien, zur automatischen, maschinell durchgeführten Krankheitserkennung, zur Erkennung einer Sepsis oder in weiteren Gebieten eingesetzt werden. Darüber hinaus erlaubt die erfindungsgemäße Möglichkeit der Sortierung von biologischen Objekten wie hierin beschrieben, eine räumliche Separierung von biologischen Objekten mit spezifischen Reaktionsmustern auf die Gabe einer Substanz, z.B. in mikrofluiden System wie hierin beschrieben. Dadurch ist eine Zuordnung von biologischen Objekten zu den aufgefundenen Analysewerten möglich. Besonders vorteilhaft ist in diesem Zusammenhang die beibehaltene Integrität und Vitalität der biologischen Objekte, z.B. Zellen, die einer zerstörungsfreien Analyse unterworfen worden sind. Somit können, in speziellen Ausführungsformen, biologische Objekte, z.B. Zellen, identifiziert, sortiert und anschließend einer weiteren Kultivierung oder Expansion oder weiteren Analyse zugeführt werden.

### KURZE BESCHREIBUNG DER FIGUREN

Die Erfindung wird nachfolgend unter Bezugnahme auf die Zeichnung anhand bevorzugter Ausführungsbeispiele weiter erläutert.
Figur 1 zeigt eine schematische Darstellung einer Vorrichtung nach einem Ausführungsbeispiel.
Figur 2 zeigt biologische Objekte an einer Arretiereinrichtung nach einem Beispiel, das nicht unter die Ansprüche fällt.
Figur 3 zeigt biologische Objekte an einer Arretiereinrichtung einer Vorrichtung nach einem Ausführungsbeispiel.
Figur 4 ist eine Draufsicht der Arretiereinrichtung von Figur 3.
Figur 5 zeigt biologische Objekte an einer Arretiereinrichtung einer Vorrichtung nach einem Ausführungsbeispiel.
Figur 6 ist eine Darstellung einer Arretiereinrichtung und einer Zuführeinrichtung einer Vorrichtung nach einem Ausführungsbeispiel.
Figur 7 ist eine Darstellung einer Arretiereinrichtung und einer Zuführeinrichtung einer Vorrichtung nach einem Ausführungsbeispiel.
Figur 8 ist eine Darstellung einer Arretiereinrichtung und einer Zuführeinrichtung einer Vorrichtung nach einem Ausführungsbeispiel.
Figur 9 zeigt biologische Objekte an einer Arretiereinrichtung einer Vorrichtung nach einem Ausführungsbeispiel.
Figur 10 zeigt biologische Objekte an einer Arretiereinrichtung einer Vorrichtung nach einem Ausführungsbeispiel.
Figur 11 zeigt biologische Objekte an einer Arretiereinrichtung einer Vorrichtung nach einem Beispiel, das nicht unter die Ansprüche fällt.
Figur 12 ist eine Draufsicht der Arretiereinrichtung von Figur 11.
Figur 13 ist eine Draufsicht einer Arretiereinrichtung einer Vorrichtung nach einem Beispiel, das nicht unter die Ansprüche fällt.
Figur 14 illustriert die Auswertung von Raman-Spektren zum Analysieren eine Reaktion eines biologischen Objekts auf eine Substanz nach einem Ausführungsbeispiel.
Figur 15 illustriert Resultate einer Hauptkomponentenanalyse bei einer Auswertung von Raman-Spektren zum Analysieren einer Reaktion eines biologischen Objekts auf eine Substanz nach einem Ausführungsbeispiel.
Figur 16 illustriert Resultate einer Hauptkomponentenanalyse bei einer Auswertung von Raman-Spektren zum Analysieren einer Reaktion eines biologischen Objekts auf eine Substanz nach einem Ausführungsbeispiel.
Figur 17 illustriert eine Clusteranalyse von Raman-Spektren zum Analysieren einer Reaktion eines biologischen Objekts auf eine Substanz nach einem Ausführungsbeispiel.
Figur 18 ist ein Flussdiagramm eines Verfahrens nach einem Ausführungsbeispiel.
Figur 19 ist ein Flussdiagramm eines Verfahrens nach einem Ausführungsbeispiel.
Figur 20 ist ein Flussdiagramm eines Verfahrens nach einem Ausführungsbeispiel.
Figur 21 ist ein Flussdiagramm eines Verfahrens nach einem Ausführungsbeispiel.
Figur 22 ist ein Flussdiagramm eines Verfahrens nach einem Ausführungsbeispiel.
Figur 23 ist ein Flussdiagramm eines Verfahrens nach einem Ausführungsbeispiel.
Figur 24 zeigt eine schematische Schnittansicht einer Arretiereinrichtung sowie die Aufteilung eines Anregungsstrahls einer Vorrichtung nach einem Ausführungsbeispiel.
Figur 25 zeigt eine schematische Schnittansicht einer Arretiereinrichtung sowie die Aufteilung eines Anregungsstrahls einer Vorrichtung nach einem Ausführungsbeispiel.
Figur 26 zeigt eine schematische Schnittansicht einer Arretiereinrichtung sowie die Aufteilung eines Anregungsstrahls einer Vorrichtung nach einem Ausführungsbeispiel.
Figur 27 illustriert einen Detektor eines Raman-Spektroskopiesystems nach einem Ausführungsbeispiel.
Figur 28 zeigt eine schematische Draufsicht einer Arretiereinrichtung einer Vorrichtung nach einem Ausführungsbeispiel.
Figur 29 zeigt eine Arretiereinrichtung, welche Microwells zum Aufnehmen biologischer Objekte in verästelnder Ausgestaltung mit zu- bzw. abführenden Kanälen aufweist. In dieser Ausführungsform können biologische Objekte nach Bedarf in einen der Kanäle verschoben, dort abtransportiert, in einem Bereich gesammelt und so sortiert werden.

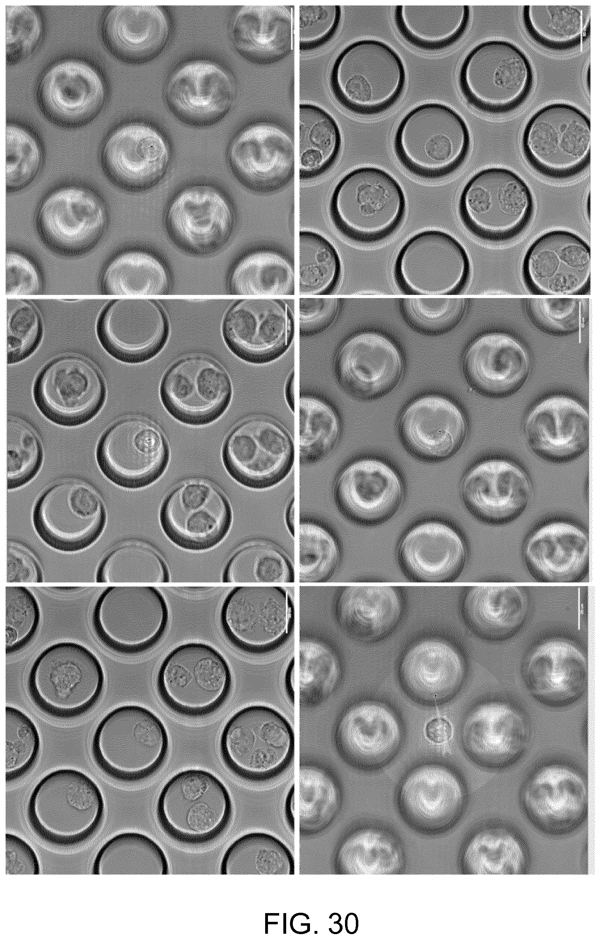

Figur 30 zeigt Microwells als Teil einer Microwellplatte in unterschiedlichen Analysestadien. Die Microwells enthalten biologische Objekte, welche Wirkstoffen ausgesetzt worden sind.

### BESCHREIBUNG VON AUSFÜHRUNGSBEISPIELEN

Ausführungsbeispiele werden unter Bezugnahme auf die Figuren beschrieben, in denen ähnliche Bezugszeichen ähnliche Merkmale bezeichnen. Die Merkmale der verschiedenen beschriebenen Ausführungsformen können miteinander kombiniert werden, sofern dies in der nachfolgenden Beschreibung nicht ausdrücklich ausgeschlossen ist.

Vorrichtungen und Verfahren nach Ausführungsbeispielen können zur Untersuchung biologischer Objekte eingesetzt werden, die von einer Arretiereinrichtung wenigstens vorübergehend arretiert werden. Vorrichtungen und Verfahren nach Ausführungsbeispielen können insbesondere eingesetzt werden, um eine Reaktion derartiger biologischer Objekte auf Substanzen wie beispielsweise Wirkstoffe, Toxine oder Chemikalien zu erfassen und automatisch auszuwerten.

Die Vorrichtungen und Verfahren nach Ausführungsbeispielen können beispielsweise zur Beobachtung von Zelldifferenzierung, zum Erkennen von Wirkstoffresistenzen, zur automatischen oder computerassistierten Entwicklung personalisierter Therapien, zur computerassistierten Erkennung von pathologischen Zuständen oder zu anderen Zwecken eingesetzt werden.

Bei Vorrichtungen und Verfahren nach Ausführungsbeispielen wird wenigstens ein Raman-Spektrum eines biologischen Objekts erfasst. Das biologische Objekt kann beispielsweise eine Zelle, einen Zellklon, ein Bakterium oder ein anderes biologisches Objekt umfassen. Es können an jedem von mehreren biologischen Objekten jeweils wenigstens ein Raman-Spektrum vor Gabe einer Substanz und wenigstens ein weiteres Raman-Spektrum während oder nach Gabe der jeweiligen Substanz erfasst werden. Durch eine statistische Auswertung eines oder mehrerer Raman-Spektren kann erkannt werden, wie das biologische Objekt auf die entsprechende Substanz reagiert.

Unterschiedliche biologischen Objekten, für die zugeordnete Raman-Spektren erfasst werden, können unterschiedlichen Substanzen ausgesetzt werden, um jeweils die Reaktion auf die entsprechende Substanz zu ermitteln. Dabei kann parallel die Reaktion mehrerer biologischer Objekte auf mehrere unterschiedliche Substanzen unter Verwendung von Raman-Spektroskopie überwacht werden.

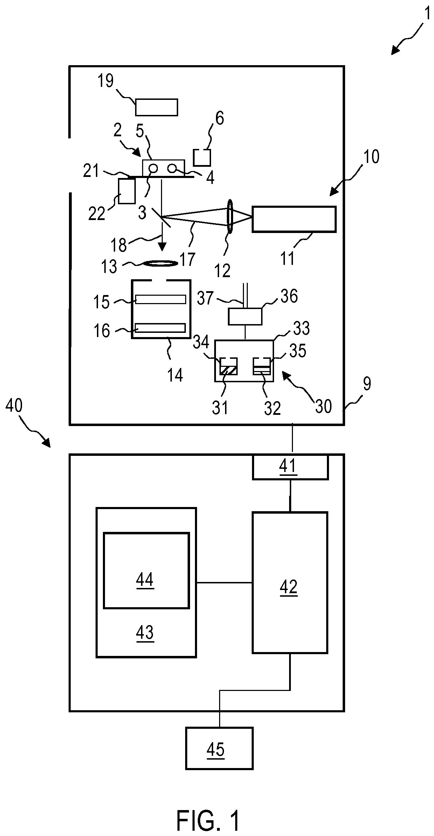

Figur 1 ist eine schematische Darstellung einer Vorrichtung 1 nach einem Ausführungsbeispiel. Die Vorrichtung 1 ist eingerichtet, um biologische Objekte 3, 4 einer Probe 2 mit Raman-Spektroskopie zu untersuchen. Die biologischen Objekte 3, 4 werden während einer Messung wenigstens vorübergehend durch eine Arretiereinrichtung 5 arretiert. Unter einer "Arretierung" wird hier allgemein verstanden, dass das biologische Objekt örtlich lokalisiert ist. Das biologische Objekt 3, 4 kann, muss jedoch nicht immobilisiert sein. Die Lokalisierung erfolgt in einer Vertiefung einer Microslide oder einer Microwellplatte, die geringfügig größer als das biologische Objekt 3, 4 ist.

Die Vorrichtung 1 kann eingerichtet sein, um die Reaktion eines oder mehrerer biologischer Objekte 3, 4, die von der Arretiereinrichtung 5 arretiert werden, auf eine Substanz oder mehrere unterschiedliche Substanzen zu erfassen und automatisch auszuwerten. Die Vorrichtung 1 kann eine Zuführeinrichtung 30 umfassen, die eingerichtet ist, um ein oder mehrere Substanzen einem oder mehreren der biologischen Objekte 3,4 zuzuführen.

Die Vorrichtung 1 umfasst ein Raman-Spektroskopiesystem 10 und eine Recheneinrichtung 40. Das Raman-Spektroskopiesystem 10 ist eingerichtet, um ein Raman-Spektrum eines biologischen Objekts oder mehrerer biologischer Objekte 3, 4 zu erfassen, die von der Arretiereinrichtung 5 arretiert werden. Die biologischen Objekte 3, 4 können jeweils ausgewählt sein aus einer Zelle, einem Zellklon, einem Bakterium und anderen biologischen Objekten.

Die Vorrichtung 1 kann optional ein Mikroskopiesystem zum Auffinden und/oder Beobachtung von mikroskopisch kleinen Objekten umfassen. Optional kann die Vorrichtung 1 eine Fluoreszenzeinheit umfassen, beispielsweise um eine Eigenfluoreszenz von biologischen Objekten anzuregen.

Das Raman-Spektroskopiesystem 10 umfasst einen Lichtquelle 11. Die Lichtquelle 11 kann ein Laser sein kann. Der Laser kann eine zellschonende Laserwellenlänge aufweisen. Die Laserwellenlänge kann 785 nm betragen. Die Lichtquelle 11 ist eingerichtet, um einen Anregungsstrahl 17 auszugeben. Ein Ramanspektrometer 14 empfängt an einem oder mehreren der biologischen Objekte 3, 4 durch Stokes-Prozesse und/oder Anti-Stokes-Prozesse gestreutes Licht 18. Das Ramanspektrometer 14 kann ein diffraktives Element 15 und einen Bildsensor 16 umfassen, um das Raman-Spektrum eines biologischen Objekts 3, 4 zu erfassen. Das Raman-Spektroskopiesystem 10 kann in an sich bekannter Weise weitere Elemente umfassen, beispielsweise fokussierende optische Elemente 12, 13, die als Linsen ausgestaltet sein können, und/oder Blenden.

Die Lichtquelle 11 kann den Anregungsstrahl mit einer Wellenlänge im Nahinfrarotbereich ausgeben. Die Lichtquelle 11 kann den Anregungsstrahl mit einer Wellenlänge ausgeben, die die Untersuchung von nativen Zellen mithilfe von Raman-Spektroskopie erlaubt.

Der Anregungsstrahl 17 kann derart durch ein Objektiv fokussiert werden dass ein elektromagnetischer Gradient entsteht, mittels welchem biologische Objekte in den Laserfokus gezogen und dort festgehalten werden können. Die kann simultan zur Spektrenerfassung geschehen, d.h. die Objekte werden während der Raman-Streuung im Laserfokus arretiert. Die optischen Haltekräfte können aber auch unabhängig von der Erfassung von Raman-Spektren eingesetzt werden, beispielsweise um die biologischen Objekte von einem zu einem andern Ort zu transportieren.

Die Vorrichtung 1 umfasst eine Recheneinrichtung 40. Die Recheneinrichtung 40 kann ein Computer sein oder kann einen Computer umfassen. Die Recheneinrichtung 40 ist mit dem Raman-Spektroskopiesystem 10 gekoppelt. Die Recheneinrichtung 40kann die Erfassung des Raman-Spektrums durch das Raman-Spektroskopiesystem 10 steuern.

Die Recheneinrichtung 40 kann die Lichtquelle 11 so steuern dass er einmal zum Erfassen von Raman Spektren eingesetzt werden kann und dass dabei simultan die biologischen Objekte im Laserfokus gehalten werden. Die Lichtquelle 11 kann aber auch so angesteuert werden, dass er unabhängig von der Erfassung eines Raman Spektrums biologische Objekte halten und/oder transportieren kann.

Die Recheneinrichtung 40 kann das Raman-Spektroskopiesystem 10 so steuern, dass Raman-Spektren ortsaufgelöst an den von der Arretiereinrichtung 5 arretierten biologischen Objekten 3, 4 erfolgt. Unterschiedliche biologische Objekte 3, 4 können zeitsequentiell einer Raman-Spektroskopie unterzogen werden, um beispielsweise ihre Reaktion auf eine Substanz zu erfassen. Die Ansteuerung, mit der die unterschiedlichen Objekte zeitsequentiell einer Raman-Spektroskopie unterzogen werden, kann automatisiert erfolgen.

Die Recheneinrichtung 40 weist eine Schnittstelle 41 auf, um Daten von dem Bildsensor 16 des Raman-Spektroskopiesystems 10 zu empfangen. Die Recheneinrichtung 40 weist eine integrierte Halbleiterschaltung 42 auf, die einen Prozessor oder Controller umfassen kann und die eingerichtet ist, um das erfasste Raman-Spektrum auszuwerten. Die integrierte Halbleiterschaltung 42 ist eingerichtet, um das wenigstens eine Raman-Spektrum auszuwerten. Die integrierte Halbleiterschaltung 42 kann beispielsweise mehrere erfasste Raman-Spektren jeweils einer statistischen Analyse unterziehen, während oder nachdem die biologischen Objekte einer Substanz ausgesetzt werden. Die statistischen Analysen können eine Hauptkomponentenanalyse, eine Clusteranalyse und/oder eine lineare Diskriminanzanalyse (LDA-Analyse) umfassen.

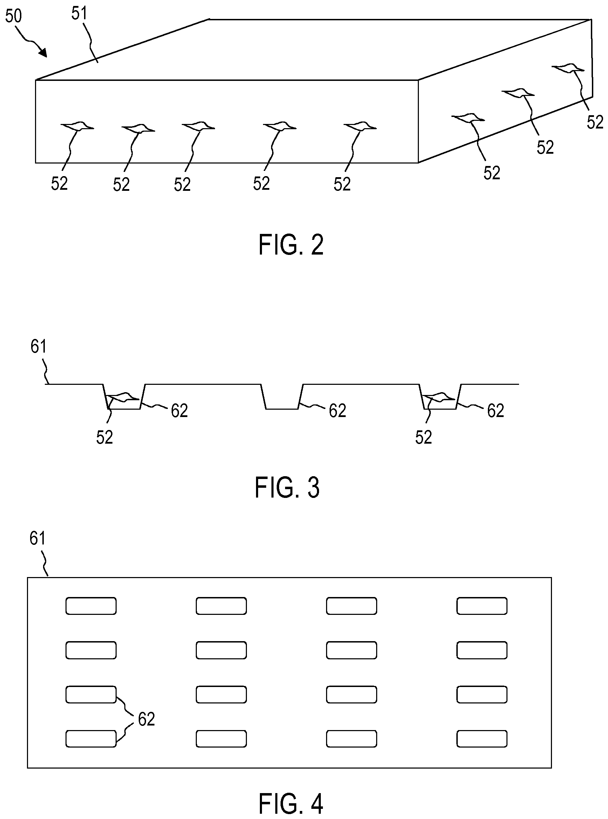

Wie unter Bezugnahme auf Figur 2 bis Figur 23 ausführlicher beschrieben wird, kann die integrierte Halbleiterschaltung 42 eingerichtet sein, um die Anwesenheit oder Abwesenheit bestimmter Raman-Peaks zu erkennen oder das spektrale Gewicht von Raman-Peaks zu bestimmen, die mit bestimmten Zellreaktionen zusammenhängen. Die integrierte Halbleiterschaltung 42 kann beispielsweise eingerichtet sein, um durch Auswertung des wenigstens einen Raman-Spektrums quantitativ zu ermitteln, ob Zellen, Zellklone, Bakterien, Keime oder andere biologische Objekte als Reaktion auf eine Substanz funktionelle Veränderungen aufweisen, beispielsweise in Apoptose, Nekrose oder einen anderen funktionellen Zustand aufweisen.

Die integrierte Halbleiterschaltung 42 kann eingerichtet sein, um für ein oder mehrere biologische Objekte jeweils zeitabhängig die Reaktion auf die diesem biologischen Objekt jeweils zugeführte Substanz auszuwerten. Auf diese Weise kann die Reaktion mehrerer biologischer Objekte parallel teil- oder vollautomatisiert zeitabhängig überwacht werden.

Die integrierte Halbleiterschaltung 42 kann unterschiedliche Zelltypen, beispielsweise unterschiedliche Blutzellen, anhand der Lage von Raman-Peaks, der Peakhöhe, der Steilheit der Flanken sowie der charakteristischen Verteilung der Peaks (Peak-Muster) für den entsprechenden Zelltyp erkennen. Information über die Lage und/oder das spektrale Gewicht unterschiedlicher Raman-Peaks für die unterschiedlichen Zelltypen können nichtflüchtig in einem Speicher der Vorrichtung 1 gespeichert sein. Alternativ oder zusätzlich kann die Information über die Lage und/oder das spektrale Gewicht unterschiedlicher Raman-Peaks für die unterschiedlichen Zelltypen, unterschiedliche Bakterien, unterschiedliche Keime oder andere biologische Objekte von der Vorrichtung 1 durch Methoden des beaufsichtigten Lernens ("Supervised Learning") oder anderer Techniken des maschinellen Lernens ermittelt werden.

Die integrierte Halbleiterschaltung 42 kann erfasste Raman-Spektren auf unterschiedliche Weise verarbeiten. Beispielsweise können statistische Methoden, z.B. eine Hauptkomponentenanalyse, Clusteranalysetechniken oder LDA-Techniken verwendet werden. Zusätzlich oder alternativ können Raman-Spektren mit Referenzdaten verglichen werden, um zu bestimmen, welche Zelltypen vorhanden sind und um die Reaktionen der Zelltypen auf unterschiedliche Substanzen zu ermitteln.

Die Recheneinrichtung 40 kann einen Speicher 43 umfassen, in dem die Referenzdaten 44 hinterlegt sind, die die integrierte Halbleiterschaltung 42 bei der Auswertung des Raman-Spektrums mit verwenden kann.

Die Recheneinrichtung 40 kann eine optische und/oder akustische Ausgabeeinheit 45 umfassen, über die abhängig von der Analyse des wenigstens einen Raman-Spektrums Information ausgegeben wird. Die Vorrichtung 1 kann eingerichtet sein, um über die optische und/oder akustische Ausgabeeinheit 45 Informationen auszugeben, die Wirkstoffresistenzen gegenüber einen oder mehreren Wirkstoffen, Verträglichkeiten einer oder mehrerer Chemikalien und/oder die Wirkung einer oder mehreren toxischen Substanzen angibt.

Die Vorrichtung 1 kann eine Bilderfassungseinrichtung 19 aufweisen. Die Bilderfassungseinrichtung 19 kann eingerichtet sein, um ein Bild der biologischen Objekte 3, 4 zu erfassen, die von der Arretiereinrichtung 5 arretiert werden. Die Bilderfassungseinrichtung 19 kann mit der integrierten Halbleiterschaltung 42 gekoppelt sein. Die integrierte Halbleiterschaltung 42 kann durch eine automatische Objekterkennung in dem erfassten Bild die biologischen Objekte 3, 4 erkennen.

Die Bilderfassungseinrichtung 19 kann so ausgestaltet sein, dass ihre Abbildungsoptik separat von einem Objektiv des Raman-Spektroskopiesystems 10 ausgebildet ist. Die Bilderfassungseinrichtung 19 kann so ausgestaltet sein, dass der optische Strahlengang von der Arretiereinrichtung 5 zu einem Bildsensor der Bilderfassungseinrichtung 19 keine optischen Komponenten des Objektivs des Raman-Spektroskopiesystems 10 durchläuft. Alternativ kann die Bilderfassungseinrichtung 19 so ausgestaltet sein, dass der optische Strahlengang von der Arretiereinrichtung 5 zu dem Bildsensor der Bilderfassungseinrichtung 19 wenigstens eine Linse des Objektivs des Raman-Spektroskopiesystems 10 durchläuft.

Die integrierte Halbleiterschaltung 42 kann einen Aktor 22 abhängig von dem von der Bilderfassungseinrichtung 19 erfassten Bild ansteuern, um sequentiell unterschiedliche von der Arretiereinrichtung 5 gehaltene biologische Objekte 3, 4 zur Messung durch das Raman-Spektroskopiesystem zu positionieren. Die integrierte Halbleiterschaltung 42 kann den Aktor 22 abhängig von der Relativposition zwischen zwei biologischen Objekten ansteuern, um sequentiell unterschiedliche biologische Objekte für eine Raman-Streuung zu positionieren.

Der Aktor 22 kann eingerichtet sein, um eine Relativbewegung zwischen der Arretiereinrichtung 5 und dem Raman-Spektroskopiesystem 10 hervorzurufen. Der Aktor 22 kann einen Motor oder einen anderen Antrieb, beispielsweise einen piezoelektrischen Antrieb, umfassen, um die Relativbewegung herbeizuführen. Der Aktor 22 kann eingerichtet sein, um die Relativbewegung in zwei und bevorzugt in drei zueinander orthogonalen räumlichen Richtungen hervorzurufen.

Der Aktor 22 kann eingerichtet sein, um einen Träger 21, an dem die Arretiereinrichtung 5 angebracht ist, relativ zu dem Raman-Spektroskopiesystem 10 zu bewegen. Der Aktor 22 kann alternativ oder zusätzlich eingerichtet sein, um wenigstens eine optische Komponente des Raman-Spektroskopiesystems 10 relativ zu dem Träger 21 zu bewegen. Der Träger 21 kann ein Mikroskoptisch sein.

Die Vorrichtung 1 umfasst eine Zuführeinrichtung 30 zum Zuführen wenigstens einer Substanz. Die Zuführeinrichtung 30 kann eingerichtet sein, um mehrere unterschiedliche Substanzen unterschiedlichen biologischen Objekten 3, 4 zuzuführen. Die Zuführeinrichtung 30 kann ein Reservoir 34 für eine Substanz 31 umfassen. Die Substanz 31 kann einen Wirkstoff, ein Toxin, eine Chemikalie oder eine andere Substanz umfassen: Die Substanz 31 kann in Lösung vorliegen. Eine Fördereinrichtung 36 kann eingerichtet sein, um die Substanz 31 aus dem Reservoir 34 zu der Arretiereinrichtung 5 zu fördern. Die Fördereinrichtung 36 kann eine Pumpe oder eine Mehrzahl von Pumpen umfassen, um unterschiedliche Substanzen unabhängig voneinander zu fördern. Die Fördereinrichtung 30 kann die Substanz 31 über eine Leitung 37 zu einem Zuführorgan fördern, das die Substanz 31 einem biologischen Objekt 3 zuführt. Die Fördereinrichtung 30 kann eingerichtet sein, um die Substanz 31 selektiv nur genau einem biologischen Objekt oder nur einer Gruppe von biologischen Objekten zuzuführen, so dass die anderen biologischen Objekte nicht der Substanz 31 ausgesetzt werden.

Die Zuführeinrichtung 30 kann ein weiteres Reservoir 35 für eine weitere Substanz 32 umfassen. Die weitere Substanz 32 kann von der Substanz 31 verschieden oder gleich der Substanz 31 sein. Die weitere Substanz 32 kann einen Wirkstoff, ein Toxin, eine Chemikalie oder eine andere Substanz umfassen: Die weitere Substanz 32 1 kann in Lösung vorliegen. Die Fördereinrichtung 36 kann eingerichtet sein, um die weitere Substanz 32 aus dem weiteren Reservoir 35 zu der Arretiereinrichtung 5 zu fördern. Die Fördereinrichtung 36 eingerichtet sein, um einen Volumenstrom der weiteren Substanz 32 unabhängig von einem Volumenstrom der Substanz 31 zu der Arretiereinrichtung 5 einzustellen. Die Fördereinrichtung 30 kann die weitere Substanz 32 über die Leitung 37 oder eine davon verschiedene Leitung zu einem Zuführorgan fördern, das die weitere Substanz 32 1 einem biologischen Objekt 4 zuführt. Die Fördereinrichtung 30 kann eingerichtet sein, um die weitere Substanz 32 selektiv nur genau einem biologischen Objekt oder nur einer Gruppe von biologischen Objekten zuzuführen, so dass die anderen biologischen Objekte nicht der weiteren Substanz 32 ausgesetzt werden.

Die Leitung 37 kann mehrere unterschiedliche Lumina umfassen. Die Leitung 37 kann eine mehrlumige Leitung sein, bei der unterschiedliche Lumina in einem Schlauch integriert sind, um unterschiedliche Substanzen 31, 34 über denselben Schlauch zu gleichen oder verschiedenen biologischen Objekten 3, 4 zu fördern.

Anstelle der Leitung 37 oder zusätzlich zu der Leitung 37 kann ein Kanal verwendet werden, in dem die Substanzen transportiert werden. Eine laminare Strömung in dem Kanal kann die Substanzen transportieren.

Auch wenn das Raman-Spektroskopiesystem 10 und die Zuführeinrichtung 30 in Figur 1 schematisch als baulich integrale Einheit dargestellt sind, kann wenigstens ein Teil der Zuführeinrichtung 30 auch separat von einem Gehäuse des Raman-Spektroskopiesystems 10 vorgesehen sein. Beispielsweise kann das Reservoir 34 und/oder das weitere Reservoir 35, die in einem Vorratsbehälter 33 ausgebildet sind, separat von dem Gehäuse des Raman-Spektroskopiesystems 10 vorgesehen sein.

Die Vorrichtung 1 kann einen Sammelbereich 6 umfassen, in dem die mithilfe einer optischen Pinzette oder durch andere Einrichtungen aus Microwells eines Mikrofluidikchips hinaustransportierten und beispielsweise in einen Fluidkanal oder Fluidstrom verbrachten Objekte gesammelt werden können.

Das Reservoir 34 und/oder das weitere Reservoir 35 können reversibel zerstörungsfrei lösbar in den Vorratsbehälter 33 einfügbar und aus dem Vorratsbehälter 33 entfernbar sein.

Auch wenn die Recheneinrichtung 40 und das Raman-Spektroskopiesystem 10 in Figur 1 schematisch als separate Einheiten dargestellt sind, können die Funktionen der Recheneinrichtung 40 auch in einem Gehäuse des Raman-Spektroskopiesystems 10 integriert sein. Das Raman-Spektroskopiesystem 10 und die Recheneinrichtung 40 können als mobile, insbesondere als tragbare Einheiten ausgestaltet sein.

Die Vorrichtung 1 kann bei Verfahren nach Ausführungsbeispielen in einer Vielzahl unterschiedlicher Weisen eingesetzt und insbesondere mit einer Vielzahl unterschiedlicher Proben 2 verwendet werden.

Die Arretiereinrichtung 5 kann eine Mikrostruktur zur Anordnung biologischer Objekte, beispielsweise Zellen und/oder Bakterien, sein.

Die Bildaufnahmeeinrichtung 19 kann ein großflächiges Übersichtsbild erfassen, das automatisch ausgewertet wird, um zu erkennen, ob biologische Objekte, beispielsweise Zellen und/oder Bakterien, an der Arretiereinrichtung 5 arretiert sind.

Die Recheneinrichtung 40 kann beispielsweise Kontrastinformation, Dunkelfeldinformation, Fluoreszenz und/oder Eigenfluoreszenz auswerten, um die Positionen biologischer Objekte zu bestimmen.

Die Mikrostruktur erlaubt ein schnelles automatisches Abtasten unterschiedlicher biologischer Objekte. So genannte Mikrowells, d.h. Vertiefungen, mit Zellen oder anderen biologischen Objekten werden abgespeichert. Raman-Spektren werden erfasst. Da die biologischen Objekte in den Mikrowells gehalten werden, beispielsweise durch Schwerkraft, Adhäsion oder ein Maschenwerk, können sie nach der Gabe des Wirkstoffes und zu bestimmten Zeiten automatisch angefahren und gemessen werden.

Die Vorrichtung 1 kann eine Spektrendatenbank aufweisen. Die Recheneinrichtung 40 kann die erfassten Spektren automatisch nach den Orten sortieren, beispielsweise nach den Mikrowells, in denen biologische Objekte enthalten sind. Es kann ein kinetischer Verlauf der Reaktion biologischer Objekte auf eine oder mehrer unterschiedliche Substanzen erfasst werden. Wie noch ausführlicher beschrieben wird, kann zur Erfassung des kinetischen Verlaufs der Reaktion eine statistische Auswertung der Raman-Spektren erfolgen, beispielsweise durch eine Hauptkomponentenanalyse, eine Clusteranalyse und/oder eine LDA.

Die Recheneinrichtung 40 kann eingerichtet sein, um eine Spezies des biologischen Objekts zu ermitteln, beispielsweise einen Zelltyp. Dazu kann die Recheneinrichtung 40 einen Abgleich mit zuvor hinterlegten charakteristischen Spektren vornehmen. Auch der Abgleich kann eine durch eine statistische Auswertung der Raman-Spektren erfolgen, beispielsweise durch eine Hauptkomponentenanalyse, eine Clusteranalyse und/oder eine LDA erfolgen.

Die Vorrichtung 1 kann für eine Parallelisierung der Analyse biologischer Objekte und/oder ihrer Reaktion auf Wirkstoffe, Toxine, Chemikalien oder andere Substanzen eingerichtet sein. Die Vorrichtung 1 kann beispielsweise Zeiten, zu denen gerade einem biologischen Objekt ein Wirkstoff zugeführt wird, verwenden, um ein Raman-Spektrum eines weiteren biologischen Objekts zu erfassen und optional auch auszuwerten, das nicht durch ein Zuführorgan überdeckt ist.

Eine Arretiereinrichtung 5, die als Micromatrix ausgestaltet ist, kann zum multiplexierten Analysieren von Zellen, Zellhaufen oder Zellklonen mittels Raman-Spektroskopie eingesetzt werden. Die Größe von Microwells bestimmt dabei, ob nur eine Zelle oder ein Zellklon oder Zellhaufen, der durch Vervielfältigung einer einzelnen Zelle wächst, mit der Raman-Spektroskopie untersucht wird.

Die Vorrichtung 1 kann eingerichtet sein, um Hintergrundsignale der Arretiereinrichtung 5 automatisch zu kompensieren. Dazu kann zusätzlich zu einer Erfassung eines Raman-Spektrums, das einem biologischen Objekt zugeordnet ist, der Aktor 22 so betätigt werden, dass das Raman-Spektrum der Arretiereinrichtung 5 an einer Position, an der kein biologisches Objekt vorhanden ist, erfasst wird. Durch Differenzspektrenbildung, bei der beispielsweise eine gewichtete Differenz eines Raman-Spektrums eines biologischen Objekts und des Raman-Spektrums der Arretiereinrichtung 5 bestimmt wird, können zunächst hintergrundkorrigierte Raman-Spektren gewonnen werden, die dann mit statistischen Analysetechniken wie einer Clusteranalyse, einer Hauptkomponentenanalyse und/oder eine LDA weiter analysiert werden, um eine Kinetik einer Reaktion auf eine Substanz zu ermitteln.

Die Vorrichtung 1 kann optional eingerichtet sein, um biologische Objekte zu sortieren. Dazu kann ein mikrofluidisches System verwendet werden. Die biologischen Objekte können abhängig von ihrer Reaktion auf eine oder mehrere Substanzen 31, 32 sortiert werden.

Beispielhaft wird nachfolgend eine Anwendung der Vorrichtung 1 zur Detektion von pathogenen Keimen und zur Evaluierung wirksamer Antibiotika beschrieben. Die Vorrichtung 1 kann sowohl zur Identifizierung bakterielle Erreger in der Blutbahn (Bakteriämie) als auch zur Auswertung der Wirksamkeit von Antibiotika oder anderen Wirkstoffen eingesetzt werden. Während beispielshaft die Detektion von pathogenen Keimen und die Ermittlung der Reaktion auf Wirkstoffe beschrieben wird, können auch andere Arten von Zellen untersucht werden, um die Reaktion von Zellen auf Wirkstoffe zu ermitteln.

Zur Identifizierung bakterielle Erreger in der Blutbahn kann die Recheneinrichtung 40 erfasste Raman-Spektren mit Daten von Raman-Spektren vergleichen, die für folgende Gattungen erfasst wurden: Gram-positive Staphylokokken, Streptokokken und Enterokokken sowie Gram-negative Escherichia, Klebsiella, Serratia und Pseudomonas.

Die Vorrichtung 1 ermöglicht die Kombination aus schneller Keimerkennung und Resistenzermittlung. Die Vorrichtung 1 ermöglicht patientenspezifisch das schnelle Finden eines optimal wirksamen Antibiotikums in einer automatischen oder computerunterstützten Prozedur. Der Aufenthalt bei einer Behandlung im Krankenhaus könnte deutlich verkürzt und damit auch Kosten entsprechend reduziert/eingespart werden. Zudem würde auf lange Sicht die Wahrscheinlichkeit von Resistenzentwicklungen sinken.

Hierzu können in einem ersten Schritt Raman-Spektren von lebenden Reinkulturen relevanter Erreger einer bakteriellen Sepsis gemessen, die als Raman-Datenbanken in der Recheneinrichtung 40 oder einem separaten Speicher gespeichert werden. Hierbei können sowohl die Bakterien selbst als auch deren Überstände mit Raman-Spektroskopie untersucht werden.

Alternativ oder zusätzlich können Blutproben von gesunden Menschen gemessen und mit denen von infizierten Patienten verglichen werden.

Zur nachfolgenden Identifizierung bakterielle Erreger können Proben des Blutplasmas sowie die Kolonien aus den Blutkulturen mit dem Raman-Spektroskopiesystem 10 gemessen werden. Die gewonnen Daten können mit den Spektren der Datenbanken abgeglichen, um für die einzelnen Erreger typische Erkennungsmuster zu finden.

Die Arretiereinrichtung kann so gestaltet sein, dass sie die Menge der Blutzellen eliminiert und die Keime an einer vordefinierten Stelle für die Erfassung der Raman-Spektren anreichert. Die Arretiereinrichtung kann hierzu einen gekrümmten Fluidkanal und/oder eine Membran umfassen.

Die Vorrichtung 1 kann zusätzlich zur Identifizierung der Keime auch eine Resistenztestung erlauben. Für die Resistenzermittlung und zum Finden geeigneter Wirkstoffe werden die Pathogene lebend von der Vorrichtung 1 untersucht. Es können unterschiedliche Wirkstoffe 31, 32 zugeführt werden, wobei während oder nach Zuführung der Wirkstoffe jeweils Raman-Spektren der biologischen Objekte erfasst werden, um zu ermitteln, auf welchen Wirkstoff oder welche Wirkstoffkombination sie reagieren und/oder für welchen Wirkstoff oder für welche Wirkstoffe 31, 32 Wirkstoffresistenzen vorliegen.

Die Proben 2 und die dabei verwendeten Arretiereinrichtungen 5 können unterschiedliche Ausgestaltungen aufweisen. Beispielsweise kann ausgehend von einer Blutprobe oder einem Blutabstrich das Plasma vom Serum, den zellulären Bestandteilen, durch Lyse der Zellen und/oder durch Zentrifugation oder mittels einer Membran getrennt werden. Ein Pellet mit den Bakterien, das beispielsweise als Sediment nach Zentrifugation verbleibt, oder die entsprechende Schicht nach einer Dichtegradientenzentrifugation kann auf einen Objektträger aufgestrichen, beispielsweise aufpipettiert, werden. Die Arretiereinrichtung kann die Bakterien auch über osmotische Verfahren anreichern. Nicht im Rahmen der Ansprüche kann der Objektträger mit einer Schicht aus einer feinmaschigen Matrix, einem so genannten "Scaffold", beschichtet sein. Die Matrix kann Hydrogel, Collagen oder Agar umfassen. Optional kann der ganze Objektträger zentrifugiert werden, so dass die Bakterien in die Schicht einwandern. Alternativ können die Bakterien mit dem Hydrogel, Collagen, oder Agar gemischt und in der Mischung ausplattiert werden, so dass beim Vernetzungsprozess die Bakterien im Maschenwerk arretiert werden.

Die beweglichen Keime können so in den Poren der Matrix oder des Scaffold arretiert werden, so dass sie nicht davonschwimmen können. Die Matrix als Arretiereinrichtung kann Flüssigkeit speichern, damit die Bakterien darin leben und sich vermehren können. Die Vorrichtung 1 kann eingerichtet sein, um Nährstoffe bzw. Wirkstoffe zu den Bakterien in den einen Mikroinkubatorraum zu transportieren. Beispielsweise kann die Arretiereinrichtung eine Gravierung aufweisen, um mikroskopisch die Keime besser auffinden oder nach Transfer in/vom Inkubator wieder auffinden zu können.

Optional können die Keime weiter aufkonzentriert werden. Hierzu können die Bakterien durch eine feinmaschige Matrix, beispielsweise Hydrogel oder Collagen, gepresst werden, welche die Flüssigkeit durchlässt, die Keime jedoch nicht. Zur Unterstützung der fragilen Konsistenz kann die Matrix auf einem Gitter, beispielsweise einen offenporigen Träger mit Gitterstruktur, aufgebracht sein. Das Gitter dient gleichzeitig als Muster zum erleichterten Auffinden der Keime unter einem Mikroskop oder in einem mit der Bildaufnahmeeinrichtung 19 erfassten Bild. Der Träger kann so ausgestaltet sein, dass er in eine Kulturschale mit Glasboden transferiert und von dem Raman-Spektroskopiesystem 10 vermessen werden kann.

Zum Aufkonzentrieren kann beispielsweise eine doppelstöckige Sterilfiltereinheit verwendet werden. Eine derartige Einheit kann mehrere Abschnitte aufweisen, in denen Filter mit unterschiedlichen Porengrößen verwendet werden. Zum Aufkonzentrieren kann eine Osmose verwendet werden.

Nach der Zentrifugation zur Aufkonzentration kann das Pellet oder die relevante Bande einer Dichtegradientenzentrifugation mit den Bakterien mit einem Hydrogel, Agar oder einer ähnlichen Matrix vermischt und in Mikrokanäle oder Mikrowells für eine Zellkultur gespritzt werden. Die einzelnen Mikrostrukturen können eine Höhe von 100 µm bis 3 mm aufweisen. Die einzelnen Mikrostrukturen können eine Höhe von 50 µm bis 3 mm aufweisen.

Während eines Vernetzungsvorgangs kann die Mikrokanal-Slide zentrifugiert werden, so dass sich die Bakterien mit dem noch flüssigen Hydrogel oder Agar am Boden oder in den Mikrowells absetzen können.

Das Hydrogel oder Agar erlaubt das Wachsen der Keime in den Mikrowells. Zum einen können die Keime vermessen und bestimmt werden. Gleichzeitig kann das Wachstum beobachtet werden. Wirkstoffe wie Antibiotika können dann in den verschiedenen Kanälen eingespritzt und die Reaktion der Keime darauf mit Raman Spektroskopie gemessen werden.

Nach erfolgter Aufkonzentrierung können die Keime auf oder zumindest unmittelbar unterhalb der Oberfläche der Arretiereinrichtung 5 liegen. Die Strukturen der Arretiereinrichtung 5 schränken die Motilität der Keime ein. Die Keime können über Eigenfluoreszenz, beispielsweise unter Ultraviolett (UV)-Licht, automatisch durch Auswertung des von der Bildaufnahmeeinrichtung 19 erfassten Bildes gefunden werden. Alternativ können die Keime auch durch einen Benutzer identifiziert und ihre Positionen über eine Benutzerschnittstelle eingezeichnet werden. Alternativ oder zusätzlich können die Keime über eine Bildanalyse-Software automatisch erkannt und markiert werden. Das Maschenwerk der Matrix kann so gestaltet sein, dass die Bakterien nicht einfach aus den Zwischenräumen entweichen können und sie somit leichter mit einem Laser, beispielsweise durch eine Optische Falle, gehalten und vermessen werden können. Gleichzeitig kann die Matrix so ausgestaltet sein, dass Nährstoffe in der Matrix diffundieren können um das Wachstum der Bakterien verfolgen und die Wirkung von Antibiotika oder anderen Wirkstoffen 31, 32 testen zu können.

Für eine Automatisierung der Analyse können die Mikrowells der Arretiereinrichtung automatisch optisch abgetastet und nach biologischen Objekten abgesucht werden. Raman-Spektren können mit Hilfe eines optischen Falleneffekts gemessen werden, da selbst in engmaschigen Matrizen immer noch eine gewisse Restbewegungsfreiheit der Keime bleibt, so dass diese während der Raman-Spektroskopie durch eine Optische Falle gehalten werden.

Die Raman-Anregung während der Raman-Spektroskopie kann somit in spezifischen Ausführungsformen gleichzeitig eine Optische Falle erzeugen. In solchen Ausführungsformen kann durch Einkopplungstechnik, z.B. Freistrahlführung und Verzicht auf Faserführung, sowie durch Fokussierung des Lasers, z.B. eines einzigen Lasers, durch ein Objektiv sowohl eine Raman-Anregung, als auch ein elektromagnetisches Gradientenfeld erzeugt werden. Dies führt zu einem Trapping-Effekt, d.h. es entsteht eine Optische Falle. Die vorliegende Erfindung macht sich diese Möglichkeiten zunutze, um biologische Objekte zu analysieren und zu arretieren.

In einer weiteren Ausführungsform kann die erfindungsgemäße Einrichtung so ausgestaltet sein, dass sie mehr als einen Laser, z.B. zwei Laser, umfasst. Typischerweise umfasst die Einrichtung dann mindestens einen Laser für die Raman-Anregung und mindestens einen zweiten Laser, welcher eine Optische Falle für das biologische Objekt erzeugt.

Wie nachfolgend noch ausführlicher beschrieben wird, können Raman Spektren vor der Gabe von Wirkstoffen sowie nach oder während Gabe von Wirkstoffen gemessen werden. Es ist möglich, aber nicht unbedingt erforderlich, die Messungen an den identischen biologischen Objekten 3, 4 zu wiederholen. Die Messungen können in einer Zeitreihe wiederholt werden, um zeitabhängig die Kinetik der Reaktion auf einen oder mehreren Wirkstoffe 31, 32 zu ermitteln. Die erfassten Raman-Spektren können zur Ermittlung der Kinetik der Reaktion auf unterschiedliche Weise weiter analysiert werden, wie unter Bezugnahme auf Figur 1 bis Figur 23 weiter beschrieben wird. Insbesondere kann eine vollautomatisierte und teilautomatisierte Datenauswertung vorgenommen werden, mit der die Recheneinrichtung 40 ermittelt, gegen welche Wirkstoffe 31, 32 eine Wirkstoffresistenz vorliegt und/oder welche Wirkstoffe 31, 32 für den jeweiligen Patienten eine erfolgversprechende Therapie ermöglichen.

Durch die Auswertung der Raman-Spektren kann die Reaktion biologischer Objekte 3, 4 auf die Wirkstoffe 31, 32 unmittelbar festgestellt werden. Es können durch die Vorrichtung 1 automatisch geeignete Antibiotika oder andere Wirkstoffe ermittelt werden. Falls eine Keimmischung vorliegt, können jeweils dieselben Keime oder Kolonien nach bestimmter Zeit vermessen werden.

Die Vorrichtungen und Verfahren nach Ausführungsbeispielen erlauben beispielsweise die automatische Ermittlung personalisierter Therapien für pathologische Zustände wir eine Sepsis, ohne hierauf beschränkt zu sein. Ausgehend von einem lebenden Blutausstrich oder direkt aus einem Bluttropfen kann ermittelt werden, welche Keime vorhanden sind und/oder welche Wirkstoffe eine erfolgreiche Therapie ermöglichen. Der Bluttropfen kann beispielsweise in einen speziellen Fluidikchip eingebracht werden, der zum Eliminieren der Blutzellen und zum Aufkonzentrieren der Keime eingerichtet ist, die dann durch Raman-Streuung untersucht werden können.

Durch die Vorrichtungen und Verfahren nach Ausführungsbeispielen können biologische Objekte wie Zellen, Zellhaufen, Zellklone, Keime, z.B. Bakterien, oder andere biologische Objekte markerfrei untersucht werden. Die Zellen können lebend bzw. zerstörungsfrei untersucht werden. Vorrichtungen und Verfahren können so implementiert sein, dass es keiner Vorbehandlung mit Markermolekülen bedarf. Die Reaktion auf Substanzen wie Wirkstoffe, Toxine, Chemikalien oder andere Substanzen kann markerfrei ermittelt werden, ohne hierzu einen physischen Kontakt zwischen einer Messeinrichtung und den biologischen Objekten herstellen zu müssen. Der Messdurchsatz kann im Vergleich zu anderen berührenden Messtechniken erhöht werden.

Weitere Merkmale von Vorrichtungen und Verfahren nach Ausführungsbeispielen werden unter Bezugnahme auf Figur 2 bis Figur 23 näher beschrieben.

Figur 2 zeigt schematisch eine Probe 50, die eine Arretiereinrichtung 51 aufweist. Die Arretiereinrichtung 51 ist nicht im Rahmen der Ansprüche. Die Arretiereinrichtung 51 kann ein Hydrogel, ein Collagen oder ein anderes Material umfassen. Die Arretiereinrichtung 51 kann Hohlräume zum Aufnehmen biologischer Objekte 52 aufweisen. Die Hohlräume können so ausgestaltet sein, dass sie für Nährstoffe für die biologischen Objekte 52 durchlässig sind.

Die Vorrichtung 1 kann bei Verwendung der Arretiereinrichtung 51 die Positionen der biologischen Objekte 52 in der Arretiereinrichtung 51 automatisch erfassen. Dazu kann die Recheneinrichtung 40 das von der Bilderfassungseinrichtung 19 erfasste Bild automatisch auswerten. Zur Positionserkennung kann beispielsweise Eigenfluoreszenz der biologischen Objekte 52 verwendet werden. Dazu kann die Vorrichtung 1 eine Lichtquelle zum Anregen der Eigenfluoreszenz aufweisen.

Die Vorrichtung 1 kann den Anregungslaser und den Bildsensor des Raman-Spektroskopiesystems 10 sowie den Aktor 22 abhängig von den erfassten Positionen der biologischen Objekte 52 steuern, um selektiv Raman-Spektren an unterschiedlichen biologischen Objekten 52 zu erfassen. Substanzen wie Wirkstoffe, Toxine, Chemikalien oder andere Substanzen können von der Zuführeinrichtung 30 zu der Arretiereinrichtung 51 gefördert werden, um die Reaktion der biologischen Objekte 52 auf die Substanz(en) zu beobachten.

Das Hydrogel, Collagen oder eine andere Matrix, in deren Zwischenräumen biologische Objekte 52 gehalten werden, kann zusätzlich in eine Microwellplatte oder eine andere Microslide eingebracht sein, wie sie unter Bezugnahme auf Figur 3 bis Figur 10 näher beschrieben wird.

Figur 3 zeigt schematisch eine Probe, die eine Arretiereinrichtung 61 aufweist. Die Arretiereinrichtung 61 kann in der Vorrichtung 1 verwendet werden. Die ArretierEinrichtung 61 kann als Microwellplatte oder eine andere Einrichtung ausgestaltet sein, die mehrere Aufnahmen 62 zum Aufnehmen biologischer Objekte aufweist. Die Aufnahmen 62 sind jeweils als Vertiefungen, beispielsweise als Microwells, ausgestaltet. Die Aufnahmen 62 können aber auch Bereiche mit einer Beschichtung erhöhter Zelladhäsivität sein, auf der die Zellen anhaften können. Die Beschichtung kann beispielsweise eine hydrophile Beschichtung sein.

Die Aufnahmen 62 können eine Tiefe und/oder laterale Abmessungen aufweisen, die an die Abmessungen der zu vermessenden biologischen Objekte angepasst sind. Beispielsweise können die Aufnahmen 62 so dimensioniert sein, dass jeweils nur eine Zelle des gewünschten Zelltyps in der Aufnahme 62 aufnehmbar ist. Die Aufnahmen 62 können auch zum Aufnehmen von Zellklonen oder Zellhaufen, die mehrere Zellen umfassen, dimensioniert sein.

In weiteren Ausführungsformen sind die Aufnahmen 62 als Microwells ausgestaltet und so angepasst, dass bei Einspülung von Wirkstoffen, z.B. Toxinen, biologische Objekte, wie etwa Zellen oder Zellklone, die sich innerhalb der Microwells befinden, aus diesen nicht herausgespült werden können. Eine bevorzugte Ausgestaltung der Microwells sieht vor, dass diese eine Tiefe besitzen, die mindestens ihrem Durchmesser entspricht.

Die Vorrichtung 1 kann bei Verwendung der Arretiereinrichtung 61 die Positionen der Aufnahmen 62 automatisch erfassen. Dazu kann die Recheneinrichtung 40 das von der Bilderfassungseinrichtung 19 erfasste Bild automatisch auswerten, um eine Lage und Art der Aufnahmen 62 der Arretiereinrichtung 61 zu erkennen.

Die Vorrichtung 1 kann weiterhin eingerichtet sein, um automatisch zu erkennen, in welchen der Aufnahmen 62 biologische Objekte 52 positioniert sind. Dazu kann die Durchlicht- oder Hellfeldbeleuchtung verwendet werden. Es kann aber auch beispielsweise die Eigenfluoreszenz der biologischen Objekte 52 verwendet werden. Es kann auch zusätzlich eine gezielte Beladung der Objekte mit Fluoreszenzmolekülen erfolgen, so dass gezielt fluoreszensmarkierte Zellen identifiziert werden können. Dazu kann die Vorrichtung 1 eine Lichtquelle zum Anregen der Eigenfluoreszenz oder einer Fluoreszenz aufweisen. Die Positionen der Aufnahmen 62, in denen jeweils ein biologisches Objekt positioniert ist, kann in einem Speicher der Recheneinrichtung 40 gespeichert werden. Zur optimalen Nutzung der Messzeit können das Raman-Spektroskopiesystem 10 und der Aktor 22 so angesteuert werden, dass gezielt Messungen an denjenigen Aufnahmen 62 vorgenommen werden, in denen biologische Objekte enthalten sind.

Wenigstens ein Raman-Spektrum kann an einer Aufnahme 62 erfasst werden, die kein biologisches Objekt 52 enthält, um beispielsweise durch Differenzspektrenbildung den Einfluss der Arretiereinrichtung 51 wenigstens teilweise zu kompensieren. Dazu kann das an der leeren Aufnahme 62 erfasste Raman-Spektrum von einem einem biologischen Objekt 52 zugeordneten Raman-Spektrum abgezogen werden. Eine Gewichtung kann bei der Differenzbildung vorgenommen werden, um das Hintergrundsignal der Arretiereinrichtung 62 weitgehend zu unterdrücken.

Die Vorrichtung 1 kann den Anregungslaser und den Bildsensor des Raman-Spektroskopiesystems 10 sowie den Aktor 22 abhängig von den erfassten Positionen der biologischen Objekte 52 steuern, um selektiv Raman-Spektren an unterschiedlichen biologischen Objekten 52 zu erfassen, die in Aufnahmen 62 gehalten werden. Substanzen wie Wirkstoffe, Toxine, Chemikalien oder andere Substanzen können von der Zuführeinrichtung 30 zu der Arretiereinrichtung 51 gefördert werden, um die Reaktion der biologischen Objekte 52 auf die Substanz(en) zu beobachten.

Sowohl bei der Arretiereinrichtung 51 von Figur 3 als auch bei der Arretiereinrichtung 61 von Figur 4 können die von der Arretiereinrichtung arretierten biologischen Objekte eine Restbeweglichkeit in den entsprechenden Aufnahmen behalten. Zur Durchführung einer Raman-Spektroskopie können die biologischen Objekte durch das Zusammenspiel der Arretiereinrichtung mit einem optischen Fallenpotential, das beispielsweise durch den Anregungsstrahl des Raman-Spektroskopiesystems 10 selbst bereitgestellt werden kann, an einer Position gehalten werden, um das Erfassen der Raman-Spektren zu ermöglichen oder/und die Güte der Raman-Daten zu verbessern.

Figur 4 zeigt eine Draufsicht einer Ausgestaltung der Arretiereinrichtung 62. Die Aufnahmen 62, die als Vertiefungen ausgestaltet sein können, können in einer regulären Anordnung vorgesehen sein. Kanäle können mehrere Aufnahmen verbinden, um die Zufuhr von Substanzen zu mehreren biologischen Objekten zu erleichtern, um die Reaktion auf diese Substanzen mit Raman-Spektroskopie zu erfassen.

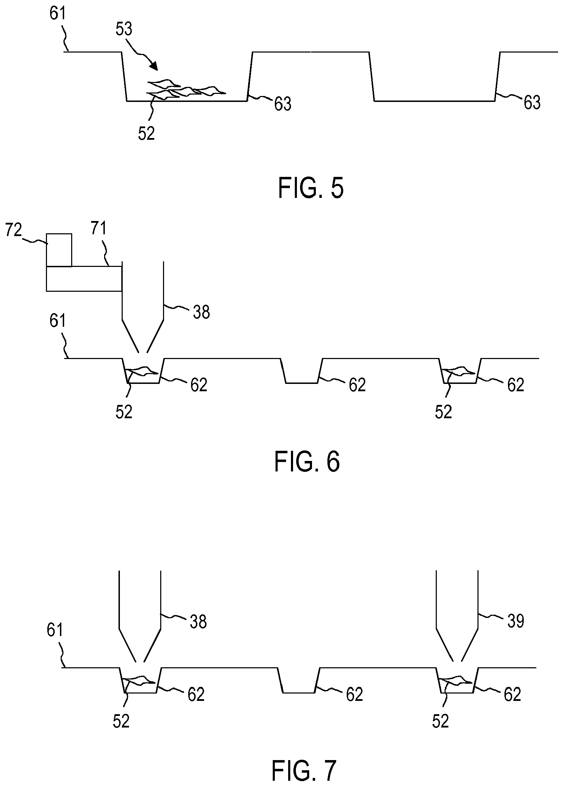

Jede Aufnahme 62 der Arretiereinrichtung 61 so ausgestaltet sein kann, dass sie nur genau ein biologisches Objekt eines bestimmten Typs, beispielsweise nur eine Zelle eines Zelltyps, aufnehmen kann. Bei anderen Ausgestaltungen können die Aufnahmen auch so ausgestaltet sein, dass sie mehrere biologische Objekte eines bestimmten Typs aufnehmen können, wie in Figur 5 dargestellt ist.

Figur 5 ist eine Schnittansicht einer Arretiereinrichtung 61 nach einem weiteren Ausführungsbeispiel. Die Arretiereinrichtung 61 kann als Microwellplatte oder eine andere Einrichtung ausgestaltet sein, die mehrere Aufnahmen 63 zum Aufnehmen biologischer Objekte aufweist. Die Aufnahmen 63 können jeweils als Vertiefungen, beispielsweise als Microwells, ausgestaltet sein.

Die Aufnahmen 63 können eine Tiefe und/oder laterale Abmessungen aufweisen, die an die Abmessungen der zu vermessenden biologischen Objekte angepasst sind. Beispielsweise können die Aufnahmen 63 so dimensioniert sein, dass jeweils nur eine Zelle des gewünschten Zelltyps in der Aufnahme 63 aufnehmbar ist. Die Aufnahmen 63 können zum Aufnehmen von Zellhaufen oder Zellklonen 53, die mehrere Zellen 52 umfassen, dimensioniert sein.

Die Aufnahmen 62, 63 der Arretiereinrichtung 61 dienen als Haltebereiche, die eingerichtet sind, um biologische Objekte wenigstens vorübergehend zu arretieren.

Figur 6 zeigt eine Teilansicht der Vorrichtung 1, die in Kombination mit jeder der verschiedenen Arretiereinrichtungen verwendet werden kann. Ein Zuführorgan 38 der Zuführeinrichtung 30 ist eingerichtet, um eine Substanz oder mehrere Substanzen einem biologischen Objekt oder mehreren biologischen Objekten zuzuführen, die von der Arretiereinrichtung arretiert werden. Das Zuführorgan 38 kann eine Düse umfassen, durch die ein Wirkstoff, ein Toxin, eine Chemikalie oder eine andere Substanz abgegeben wird. Das Zuführorgan 38 kann über die Fördereinrichtung 36 mit wenigstens einem Reservoir 34, 35 verbunden sein.

Die Zuführeinrichtung kann einen oder mehreren Mikrofluidikkanälen umfassen, durch den oder durch die eine Substanz auf eine Vielzahl von Zellen oder Zellhaufen geleitet wird. Es können mehrere Kanäle nebeneinander existieren, wobei die Zuführeinrichtung eingerichtet sein kann, um in den mehreren Kanälen gleichartige oder verschiedenartige Substanzen zu applizieren.

Die Vorrichtung 1 kann einen weiteren Aktor 72 zum Herbeirufen einer Relativbewegung zwischen der Arretiereinrichtung 61 und dem Zuführorgan 38 aufweisen. Der weitere Aktor 72 kann unabhängig von dem Aktor 22 betätigbar sein. Der weitere Aktor 72 kann eingerichtet sein, um das Zuführorgan 38 relativ zu der Arretiereinrichtung 61 zu bewegen und/oder um die Arretiereinrichtung 61 relativ zu dem Zuführorgan 38 zu bewegen. Der weitere Aktor 72 kann beispielsweise einen Halter 71 für das Zuführorgan linear in zwei oder drei orthogonalen Raumrichtungen verstellen.

Kanäle können in der Arretiereinrichtung 61 vorhanden sein, um einen Teil der Aufnahmen 62 miteinander zu verbinden, so dass die über das Zuführorgan 38 zugeführte Substanz zu biologischen Objekten in mehreren Aufnahmen 62 weitergeleitet wird. Die Statistik der Messung kann so verbessert werden.

Figur 7 zeigt eine Teilansicht der Vorrichtung 1, die in Kombination mit jeder der verschiedenen Arretiereinrichtungen verwendet werden kann. Ein Zuführorgan 38 der Zuführeinrichtung 30 ist eingerichtet, um eine Substanz oder mehrere Substanzen einem biologischen Objekt oder mehreren biologischen Objekten zuzuführen, die von der Arretiereinrichtung arretiert werden. Ein weiteres Zuführorgan 39 ist eingerichtet, um eine weitere Substanz zuzuführen. Die Zuführorgane 38, 39 können unterschiedliche Substanzen zuführen. Das Zuführorgan 38 und das weitere Zuführorgan 39 kann jeweils eine Düse umfassen, durch die ein Wirkstoff, ein Toxin, eine Chemikalie oder eine andere Substanz abgegeben wird. Das Zuführorgan 38 kann über die Fördereinrichtung 36 mit wenigstens einem Reservoir 34 verbunden sein. Das weitere Zuführorgan 39 kann über die Fördereinrichtung 36 mit wenigstens einem weiteren Reservoir 35 verbunden sein.

Die Zuführeinrichtung 30 kann eingerichtet sein, um über die Zuführorgane 38, 39 parallel mehreren unterschiedlichen biologischen Objekten dieselben oder unterschiedliche Substanzen zuzuführen. Beispielsweise können die biologischen Objekte von der Recheneinrichtung 40 oder benutzerdefiniert in mehrere Gruppen unterteilt werden, die eine erste Gruppe und eine zweite Gruppe mit jeweils einer Mehrzahl biologischer Objekte umfassen. Biologischen Objekten der ersten Gruppe kann eine erste Substanz über das Zuführorgan 38 zugeführt werden. Biologischen Objekten der zweiten Gruppe kann eine zweite Substanz über das weitere Zuführorgan 39 zugeführt werden. Die Reaktion der biologischen Objekte beider Gruppen auf die unterschiedlichen Substanzen kann jeweils durch Raman-Spektroskopie untersucht werden.

Die Arretiereinrichtung 61 kann Kanäle aufweisen, die einen Teil der Aufnahmen 62 miteinander verbinden, so dass die über das Zuführorgan 38 zugeführte Substanz zu biologischen Objekten in mehreren Aufnahmen 62 weitergeleitet wird. Die Kanäle können mehrere voneinander disjunkte Gruppen von Aufnahmen 62, beispielsweise eine erste Gruppe von miteinander verbundenen Aufnahmen und eine davon disjunkte zweite Gruppe von miteinander verbundene Aufnahmen, definieren.

Die Raman-Spektren, die biologischen Objekten zugeordnet sind, können durch Raman-Spektroskopie an den biologischen Objekten selbst erfasst werden. Alternativ oder zusätzlich können Raman-Spektren, die biologischen Objekten zugeordnet sind, auch an einem die biologischen Objekte wenigstens teilweise überdeckenden Fluid, beispielsweise einer Flüssigkeit, erfasst werden. Auf diese Weise können Metabolite oder andere von den biologischen Objekten ausgeschleuste Stoffe durch Raman-Spektroskopie erfasst werden, um die Reaktion auf eine Substanz zu ermitteln.

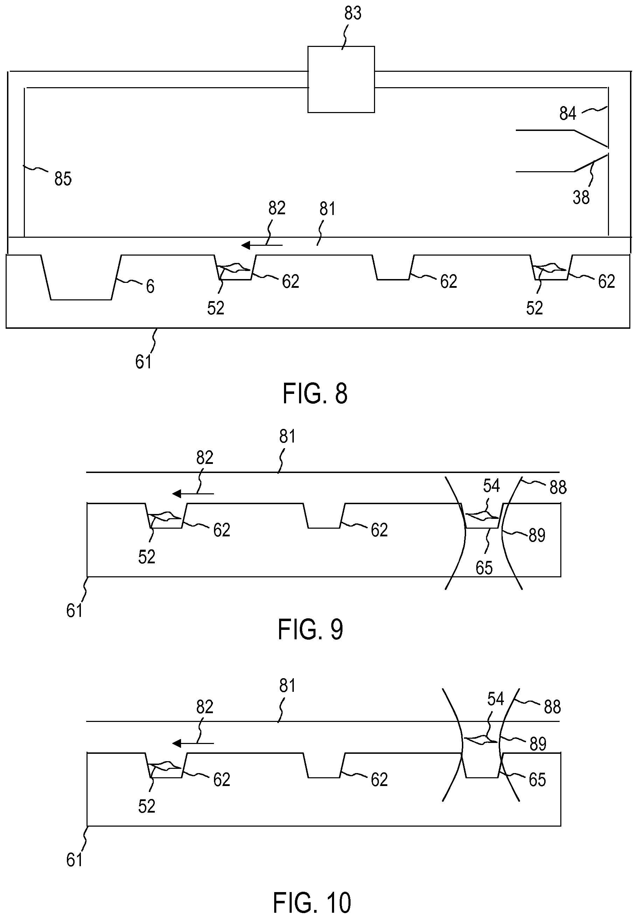

Figur 8 zeigt eine Teilansicht der Vorrichtung 1, die in Kombination mit jeder der verschiedenen Arretiereinrichtungen verwendet werden kann. Aufnahmen 62 der Arretiereinrichtung 61 sind wenigstens teilweise durch ein Fluid 81 überdeckt.

Die Arretiereinrichtung 61 kann eine Sammelaufnahme 6 oder mehrere Sammelaufnahmen umfassen. Die Sammelaufnahme(n) 6 können in dem Mikrofluidikchip ausgebildet sein. In die Sammelaufnahme(n) 6 können Zellen aus den Aufnahmen 62 über den Fluidstrom verbracht werden.

Ein Transfer von einer Aufnahme 62 in die Sammelaufnahme 6 kann das Bewegen der Zelle aus der Aufnahme 62 in den Strom des Fluids 81, ein Transportieren der Zelle in dem Strom des Fluids 81 und das Bewegen der Zelle aus dem Strom des Fluids 81 in die Sammelaufnahme 6 umfassen. Das Bewegen aus der Aufnahme 62 in den Strom des Fluids 81 und/oder das Bewegen aus dem Strom des Fluids 81 in die Sammelaufnahme 6 kann jeweils unter Verwendung optischer Mittel, insbesondere einer beweglichen optischen Falle, implementiert werden.

Eine Entscheidung, in welcher von mehreren Sammelaufnahmen eine Zelle gesammelt werden soll, kann abhängig von vorher in der Aufnahme 62 erfassten Raman-Spektren erfolgen. Alternativ oder zusätzlich kann eine Entscheidung, ob eine Zelle überhaupt in einer Sammelaufnahme 6 gesammelt werden soll, abhängig von vorher in der Aufnahme 62 erfassten Raman-Spektren erfolgen. Die entsprechende Entscheidung kann automatisch von der Vorrichtung 1 abhängig von den vorher erfassten Raman-Spektren getroffen werden.

Durch die Sammelaufnahmen 6 lassen sich Zellen abhängig von ihren Eigenschaften getrennt sammeln.

Die Vorrichtung 1 kann eingerichtet sein, um das Fluid 81 in einem Fluidstrom zu bewegen. Das Fluid 81 kann eine Flüssigkeit sein.

Die Vorrichtung 1 kann beispielsweise eine Pumpe 83 oder eine andere Einrichtung zum Erzeugen einer Strömungsgeschwindigkeit des Fluids 81 aufweisen. Wenigstens ein Kanal 84, 85 kann zum Zuführen des Fluids von der Arretiereinrichtung 61 und/oder zum Ableiten des Fluids von der Arretiereinrichtung 61 vorgesehen sein.

Biologische Objekte 52 können in dem Fluid 81 transportiert werden. Die Vorrichtung 1 kann eine Einrichtung zum selektiven Bewegen biologischer Objekte zwischen einem Fluidstrom 82, der wenigstens einen Teil der Aufnahmen 62 überströmt, und den Aufnahmen 62 aufweisen. Die Einrichtung kann eine Optische Pinzette oder andere elektrische und/oder magnetische Felder oder Wellen umfassen, mit denen biologische Objekte 52 zwischen dem Fluidstrom 82 und den Aufnahmen 62 bewegt werden.

Der Fluidstrom 82 muss nicht notwendig als ein geschlossener Fluidkreislauf implementiert sein. Beispielsweise kann der Fluidstrom 82 verwendet werden, um biologische Objekte 52 abhängig von den erfassten Raman-Spektren zu sortieren. Biologische Objekte 52 können durch eine steuerbare Einrichtung zum Kontrollieren des Fluidstroms 82 in eine Mehrzahl unterschiedlicher Behältnisse transportiert werden, abhängig davon, welche Reaktion auf eine Substanz die biologischen Objekte anhand der Raman-Spektren zeigen, und/oder abhängig von dem mittels Raman-Spektroskopie ermittelten Typus des biologischen Objekts.

Die Zufuhr einer oder mehrerer Substanzen kann in den Fluidstrom 82 integriert sein. Beispielsweise kann ein Zuführorgan 38 zum Zuführen einer oder mehrerer Substanzen eine Auslassöffnung aufweisen, die in einer Fluidverbindung mit einem Zuführkanal 84 für das Fluid 81 steht. Durch Steuerung der Fördereinrichtung 36 kann die Konzentration der Substanz(en) 31, 32 in dem Fluid 81 gesteuert oder geregelt werden.

Figur 9 und Figur 10 veranschaulichen die Funktionsweise einer Einrichtung zum selektiven Bewegen biologischer Objekte zwischen dem Fluidstrom 82 und den Aufnahmen 62. Die Einrichtung zum selektiven Bewegen biologischer Objekte kann eine Optische Pinzette umfassen. Ein Strahlungsfeld 88 kann einen Fokalbereich 89 aufweisen, der senkrecht zu einer Oberfläche der Arretiereinrichtung 61 bewegbar ist. Das Strahlungsfeld 88 kann mit dem Anregungsstrahl des Raman-Spektroskopiesystems 10 oder mit einem davon verschiedenen Laserstrahl erzeugt werden.

Durch Steuern des Strahlungsfelds 88 derart, dass der Fokalbereich 89 senkrecht zur Ebene der Arretiereinrichtung 61 bewegt wird, wird ein biologisches Objekt 54 zwischen dem Fluidstrom 82 und einer Aufnahme 65 bewegt, wie in Figur 10 dargestellt ist. In der Aufnahme 65 kann sich das biologische Objekt 54 vorher durch die auf es wirkende Schwerkraft aus dem Fluidstrom 82 abgesetzt haben. Optional kann das biologische Objekt 54 in der Aufnahme 54 durch das Strahlungsfeld 88 und/oder durch die Ränder der Aufnahme 65 gehalten werden. Das Strahlungsfeld 88 muss nicht fortgesetzt aktiviert bleiben, um das biologische Objekt 54 in der Aufnahme 65 zu halten. Das biologische Objekt kann sich beispielsweise auch unter Wirkung der Schwerkraft in der Aufnahme 65 absetzen, nachdem es mittels Fluidstrom 82 eingespült wurde.

Optional kann das biologische Objekt 54 aus dem Fluidstrom 82 unter Verwendung eines optischen oder anderen Strahlungsfelds in die Aufnahme 65 bewegt werden.

Alternativ oder zusätzlich kann das Aufnehmen von Zellen in den Aufnahmen 62, 65 dadurch erfolgen, dass die Zellen durch den Fluidikkanal eingespült werden und sich aufgrund der Schwerkraft in den Aufnahmen 62, 65 absetzen. Ein Transfer von den Aufnahmen 62, 65 in den Fluidstrom kann mit einer optischen Falle, durch andere elektromagnetische Felder oder Wellen, durch selektive Ausübung von Fluiddruck, oder auf andere Weise erfolgen. Aus dem Fluidstrom können die Zellen dann in Sammelaufnahmen des Mikrofluidikchips transferiert werden.

Anstelle oder zusätzlich zu einem optischen Strahlungsfeld 88 können andere elektrische, magnetischer oder elektromagnetische Felder oder Wellen verwendet werden, um ein biologisches Objekt 65 zwischen dem Fluidstrom 82 und einer Aufnahme 62, 65 zu bewegen. Unabhängig von der spezifischen Implementierung der Einrichtung zum selektiven Bewegen biologischer Objekte kann die Vorrichtung 1 so ausgestaltet sein, dass die biologischen Objekte zerstörungsfrei reversibel von dem Fluidstrom 82 in eine Aufnahme 65 und von der Aufnahme 65 wieder in den Fluidstrom 82 überführbar sind.

Weitere Ausgestaltungen von Arretiereinrichtungen können bei Vorrichtungen und Verfahren nach Ausführungsbeispielen verwendet werden.

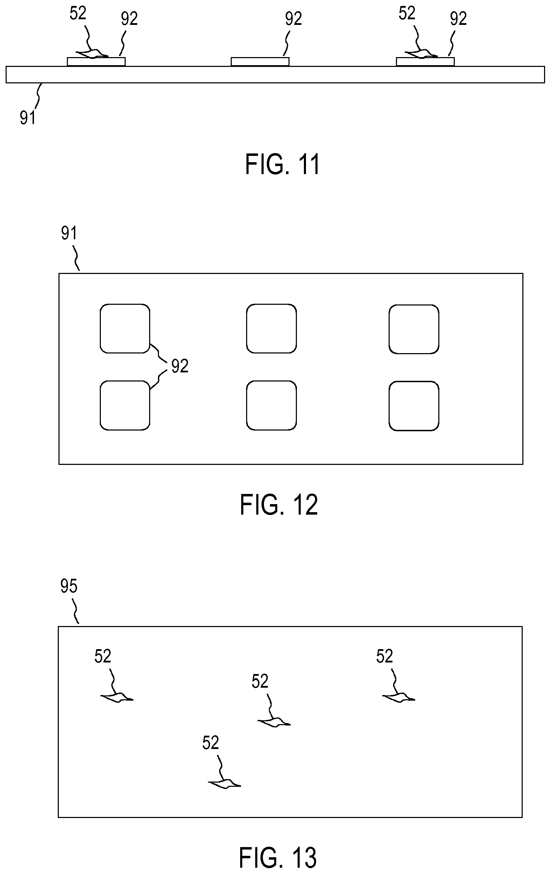

Figur 11 zeigt eine Schnittansicht einer Arretiereinrichtung 91, die nicht unter die Ansprüche fällt und Figur 12 zeigt eine Draufsicht der Arretiereinrichtung 91. Die Arretiereinrichtung 91 weist Bereiche 92 auf, die strukturiert auf einer Oberfläche der Arretiereinrichtung 91 angeordnet sind. Die Bereiche 92 können Beschichtungen sein oder eine andere Ausgestaltung aufweisen. Die Bereiche 92 sind eingerichtet, um biologische Objekte 52 wenigstens vorübergehend daran zu arretieren. Die Bereiche 92 können in einer regulären oder irregulären Anordnung vorgesehen sein.

Die Vorrichtung 1 kann bei Verwendung der Arretiereinrichtung 91 die Positionen der Bereiche 92 automatisch erfassen. Dazu kann die Recheneinrichtung 40 das von der Bilderfassungseinrichtung 19 erfasste Bild automatisch auswerten, um eine Lage und Art der Bereiche 92 der Arretiereinrichtung 91 zu erkennen.

Die Vorrichtung 1 kann weiterhin eingerichtet sein, um automatisch zu erkennen, in welchen der Bereiche 92 biologische Objekte 52 positioniert sind. Dazu kann beispielsweise ein Kontrast, Eigenfluoreszenz oder eine Fluoreszenzmarkierung der biologischen Objekte 52 verwendet werden. Dazu kann die Vorrichtung 1 eine Lichtquelle zum Anregen der Eigenfluoreszenz oder der Fluoreszenz aufweisen. Die Positionen der Bereiche 92, in denen jeweils ein biologisches Objekt positioniert ist, kann in einem Speicher der Recheneinrichtung 40 gespeichert werden. Zur optimalen Nutzung der Messzeit können das Raman-Spektroskopiesystem 10 und der Aktor 22 so angesteuert werden, dass gezielt Messungen an denjenigen Bereichen 92 vorgenommen werden, an denen biologische Objekte arretiert sind.

Wenigstens ein Raman-Spektrum kann an einem Bereich der Arretiereinrichtung 92 aufgenommen werden, der kein biologisches Objekt 52 enthält, um beispielsweise durch Differenzspektrenbildung den Einfluss der Arretiereinrichtung 91 wenigstens teilweise zu kompensieren.

Die Vorrichtung 1 kann den Anregungslaser und den Bildsensor des Raman-Spektroskopiesystems 10 sowie den Aktor 22 abhängig von den erfassten Positionen der biologischen Objekte 52 steuern, um selektiv Raman-Spektren an unterschiedlichen biologischen Objekten 52 zu erfassen, die an den Bereichen 92 arretiert werden. Substanzen wie Wirkstoffe, Toxine, Chemikalien oder andere Substanzen können von der Zuführeinrichtung 30 zu der Arretiereinrichtung 91 gefördert werden, um die Reaktion der biologischen Objekte 52 auf die Substanz(en) zu beobachten.

Figur 13 zeigt eine Arretiereinrichtung 95, die nicht unter die Ansprüche fällt, und die eine Oberfläche aufweist, an der biologische Objekte 52 adhärent anwachsen können. Die Arretiereinrichtung 95 kann eine homogene oder strukturierte Beschichtung aufweisen, die das Anwachsen der biologischen Objekte erleichtern kann.

Die Arretiereinrichtung 95 kann eine mit Zellen vermischte, beispielsweise inkubierte, Matrix sein, bei der die Zellen zufällig verteilt sind. Die Vorrichtung 1 kann die Position der Zellen über Bilderkennung automatisch auffinden. Dazu kann entweder ein Kontrast, eine Eigenfluoreszenz oder eine Fluoreszenz der Zellen ausgewertet werden.

Bei jeder der unter Bezugnahme auf Figur 2 bis Figur 13 beschriebenen Arretiereinrichtungen können biologische Objekte in einem Fluidstrom transportiert und optional sortiert werden, wie dies unter Bezugnahme auf Figur 8 bis Figur 10 beschrieben wurde.

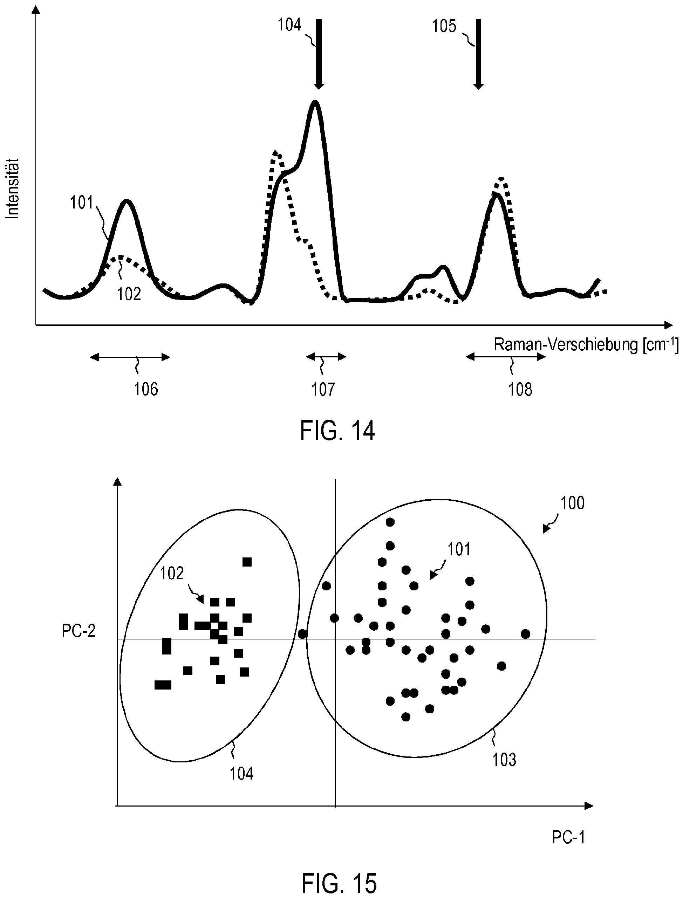

Unter Bezugnahme auf Figur 14 bis Figur 23 werden Techniken beschrieben, die von der Vorrichtung 1 automatisch ausgeführt werden können, um die Reaktion biologischer Objekte auf eine Substanz oder mehrere unterschiedliche Substanzen automatisch zu ermitteln und/oder um den Typ biologischer Objekte zu bestimmen, beispielsweise um einen Keim oder einen Zelltyp zu identifizieren.

Figur 14 zeigt Raman-Spektren 101, 102 für ein biologisches Objekt. Ein erstes Raman-Spektrum 101 kann vor Gabe einer Substanz erfasst werden. Wenigstens ein zweites Raman-Spektrum 102 kann während oder nach Gabe der Substanz erfasst werden. Anhand der Verschiebung von Wellenzahlen, an denen Raman-Peaks vorliegen, und/oder anhand dem spektralen Gewicht unterschiedlicher Raman-Peaks 104, 105 kann ermittelt werden, ob das biologische Objekt eine Reaktion auf die Substanz zeigt oder nicht. Diese Unterschiede können von der Vorrichtung 1 zur automatischen Ermittlung der Reaktion des biologischen Objekts auf die Substanz, beispielsweise zur Erkennung von Wirkstoffresistenzen, verwendet werden.

Unterschiedliche Wellenzahlen oder Wellenzahlintervalle 106, 107, 108 können ausgewertet werden, um die Reaktion des biologischen Objekts zu bestimmen. Beispielsweise können die Raman-Spektren in einem oder mehreren der Wellenzahlintervalle 813-832 cm⁻¹, 864-888 cm⁻¹, 911-920 cm⁻¹, 984-1070 cm⁻¹, 1038-1051 cm⁻¹, 1078-1091 cm⁻¹, 1100-1131 cm⁻¹ und/oder 1430-1443 cm⁻¹ ausgewertet werden, um einen Übergang von lebenden zu apoptotischen Zuständen biologischer Objekte zu erkennen. Dies kann sowohl zur Ermittlung der Verträglichkeit chemischer Substanzen als auch zur Ermittlung von Wirkstoffresistenzen relevant sein.

Es können von der Vorrichtung 1 Muster der Spektren erfasst und Unterschiede in den Mustern als Diskriminierungsgrößen verwendet und optional ermittelt werden. Beispiele für derartige Muster beinhalten eine oder mehrere Peaklagen, Peakhöhen, Steilheit der Flanken, Form und Lage der Täler zwischen den Peaks und/oder andere aus dem Spektrum ableitbare Größen.

Das Raman-Spektrum oder die Raman-Spektren können von der Recheneinrichtung 40 weiter verarbeitet werden. Die Recheneinrichtung 20 kann beispielsweise eine Clusteranalyse, eine Hauptkomponentenanalyse oder eine LDA der erfassten Raman-Spektren ausführen. Vor einer derartigen Analyse können durch Differenzspektrenbildung Signalanteile der Arretiereinrichtung wenigstens teilweise unterdrückt werden.

Das Ergebnis der Clusteranalyse kann verwendet werden, um Reaktionen und eine Geschwindigkeit von Reaktionen biologischer Objekte auf eine oder mehrere Substanzen quantitativ zu analysieren.

Figur 15 veranschaulicht beispielhafte Ergebnisse 100 einer Hauptkomponentenanalyse, die von der Recheneinrichtung 40 durchgeführt wird, um zu bestimmen, ob ein biologisches Objekt eine Reaktion auf die Gabe einer Substanz zeigt. Dabei wird die Hauptkomponentenanalyse für ein Raman-Spektrum oder mehrere Raman-Spektren ausgeführt, die an demselben biologischen Objekt 3, 4 erfasst wurden. Die Datenpunkte sind gemäß einem Paar der unterschiedliche Hauptkomponenten PC-1 und PC-2 dargestellt. Figur 15 zeigt die Datenpunkte 101, die lebenden Zellen zugeordnet sind, und Datenpunkte 102, die apoptotischen Zellen zugeordnet sind. Vergleichbare Datenpunkte können sich auch aufgrund eines veränderten funktionellen Zustandes der Zellen nach der Wirkstoffgabe ergeben. Ähnliche Strukturen ergeben sich auch bei einer Hauptkomponentenanalyse der Raman-Spektren anderer biologischer Objekte wie Keime, z.B. Bakterien.

Das Ergebnis der Hauptkomponentenanalyse der an dem biologischen Objekten erfassten Raman-Spektren kann dahingehend ausgewertet werden, ob und wie viele Datenpunkte in unterschiedlichen Regionen 103, 104 des durch mehrere Hauptkomponenten aufgespannten Koordinatensystems liegen. Beispielsweise kann ermittelt werden, wie viele Datenpunkte in einer Region 103 liegen, die lebenden Zellen eines bestimmten Zelltyps zugeordnet ist. Es kann ermittelt werden, wie viele Datenpunkte in einer Region 104 liegen, die apoptotischen Zellen desselben Zelltyps zugeordnet ist. Es kann ermittelt werden, wie viele Datenpunkte in weiteren Regionen des durch mehrere Hauptkomponenten aufgespannten Koordinatensystems liegen, die anderen biologischen Objekten und/oder anderen funktionellen Zuständen der biologischen Objekte zugeordnet sind.

Wie aus Figur 15 erkennbar, verschieben sich die durch die Hauptkomponentenanalyse gewonnenen Datenpunkte abhängig davon, ob und in welcher Weise ein biologisches Objekt auf eine Substanz reagiert. Entsprechend kann die Recheneinrichtung 40 anhand der Hauptkomponentenanalyse eines Raman-Spektrums oder mehrerer Raman-Spektren automatisch bestimmen, welche Zelltypen vorhanden sind und/oder ob die Zellen als Reaktion auf unterschiedliche Substanzen in einen apoptotischen Zustand übergehen.

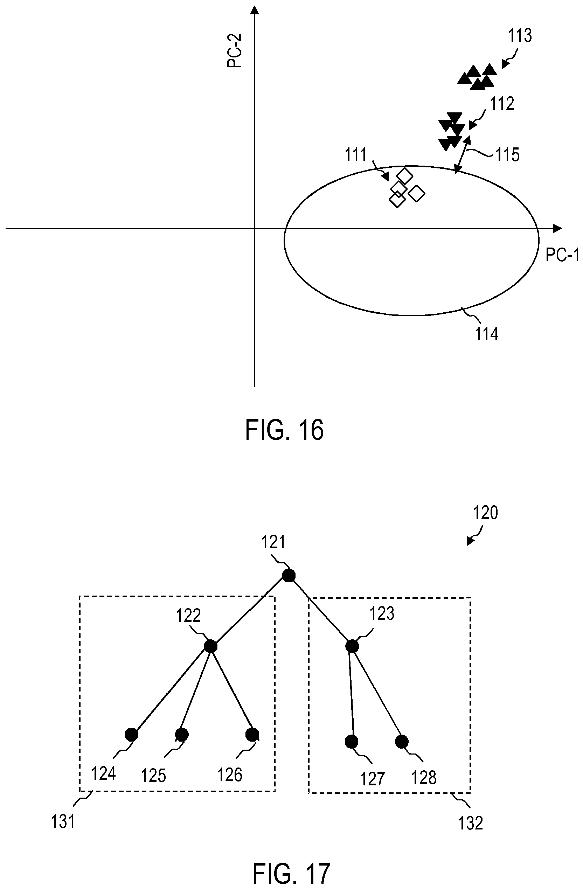

Figur 16 veranschaulicht die Auswertung einer Kinetik einer Reaktion eines biologischen Objekts auf eine Substanz. Dargestellt ist jeweils das Ergebnis einer Hauptkomponentenanalyse. Vor Gabe einer Substanz liegen die Datenpunkte 111 einer Hauptkomponentenanalyse des Raman-Spektrums eines biologischen Objekts in einem Bereich 114 des durch die Hauptkomponenten aufgespannten Raums. Der Bereich 114 entspricht funktionell intakten biologischen Objekten, z.B. funktionell intakten lebenden Zellen oder Bakterien.

Als Reaktion auf die Gabe der Substanz, beispielsweise eines Toxins oder eines Wirkstoffes, erfolgt einer Verschiebung der Datenpunkte. Nach einer ersten Zeit nach Gabe der Substanz können die Datenpunkte 112, die durch Hauptkomponentenanalyse des Raman-Spektrums eines biologischen Objekts ermittelt werden, den Bereich 114 verlassen haben. Dies zeigt an, dass eine funktionelle Veränderung, beispielsweise durch Übergang in einen apoptotischen Zustand, erfolgt.

Nach einer zweiten Zeit nach Gabe der Substanz, die länger als die erste Zeit ist, können die Datenpunkte 113, die durch Hauptkomponentenanalyse des Raman-Spektrums eines biologischen Objekts ermittelt werden, sich noch weiter von dem Bereich 114 im Hauptkomponentenraum entfernt haben.

Ein Abstand 115 zwischen den Datenpunkten 112, 113 und der Grenze des Bereichs 114 im Hauptkomponentenraum kann zeitabhängig ermittelt werden, um funktionelle Veränderungen biologischer Objekte als Reaktion auf die Gabe der entsprechenden Substanz zu ermitteln.

Eine derartige Verarbeitung kann nicht nur für eine, sondern parallel für die Gabe einer Mehrzahl unterschiedlicher Substanzen erfolgen. Dabei können unterschiedlichen biologischen Objekten vom selben Typ unterschiedliche Substanzen zugeführt werden, um aus den Raman-Spektren Information über die Kinetik der Reaktion auf die Substanz zu erhalten.

Durch Vergleich der beiden Spektren vor und nach der Gabe eines Wirkstoffes können von der Vorrichtung 1 auch die Art der Moleküle bestimmt werden, die für die Veränderung des Raman-Spektrums maßgeblich sind. Dazu können Differenzspektren, beispielsweise so genannte "Loadings", und/oder ein Abgleich mit in einer Datenbank gespeicherter Information über Spektren und/oder ein Vergleich mit in der Literatur dokumentierten Raman-Charakteristika unterschiedlicher Moleküle verwendet werden.

Andere Verarbeitungstechniken können von der Recheneinrichtung 40 automatisch angewandt werden. Beispielsweise kann eine LDA oder eine Clusteranalyse ausgeführt werden, um die Reaktion biologischer Objekte auf eine oder mehrere Substanzen zu ermitteln.
Figur 17 veranschaulicht schematisch die Funktionsweise der Recheneinrichtung 40 der Vorrichtung 1 bei einer derartigen statistischen Auswertung, die eine Clusteranalyse umfasst.

Durch Auswertung einer Mehrzahl von Raman-Spektren, die unterschiedlichen Reaktionen biologischer Objekte auf verschiedene Substanzen zugeordnet sind, wird entweder von der Recheneinrichtung 40 der Vorrichtung 1 selbst oder auch fern von der Vorrichtung 1 ein Baum 120 von Raman-Spektren aufgebaut. Der Baum 120 enthält eine Mehrzahl von Knoten 121-128. Raman-Spektren werden den Knoten 121-128 basierend auf einem Ähnlichkeitsmaß, das beispielsweise auf einer Cosinus-Entfernung beruhen kann, zugeordnet.

Der Baum 120 kann einen ersten Teilbaum 131 aufweisen, der lebenden biologischen Objekten zugeordnet ist. Der Baum 120 kann einen zweiten Teilbaum 132 aufweisen, der apoptotischen biologischen Objekten oder anderen funktionell veränderten biologischen Objekten zugeordnet ist.

Bei der Auswertung eines Raman-Spektrums oder mehrerer Raman-Spektren, die an biologischen Objekten 3, 4 während oder nach Gabe einer Substanz erfasst wurden, kann die Recheneinrichtung 40 der Vorrichtung 1 jeweils anhand des Abstandsmaßes berechnen, welchem der Blattknoten 124-128 und/oder inneren Knoten 122, 123 des Baumes 120 das entsprechende Raman-Spektrum zuzuordnen ist.

Auf diese Weise können Raman-Spektren auch ohne a priori Kenntnis einzelner relevanter Wellenzahlen verschiedenen Reaktionen auf die Gabe einer Substanz zugeordnet werden. Wenigstens einer der unterschiedlichen Blattknoten 124-128 kann beispielsweise Raman-Spektren zugeordnet sein, die an einer apoptotischen Zelle eines bestimmten Zelltyps erfasst wurden. Wenigstens ein weiterer der unterschiedlichen Blattknoten 124-128 kann beispielsweise Raman-Spektren zugeordnet sein, die an einer lebenden Zelle desselben Zelltyps erfasst wurden.

Zur Unterscheidung unterschiedlicher Reaktionen biologischer Objekte auf Substanzen kann allgemein das Raman-Spektrum jedes biologischen Objekts, an der die Messung ausgeführt wird, eine Anzahl N von Intensitäten bei unterschiedlichen Wellenlängen umfassen. Die Anzahl N kann größer als eins, insbesondere viel größer als eins sein. Durch eine hierarchische Clusterung, beispielsweise in einem N-dimensionalen Raum, kann ausgenutzt werden, dass in diesem Raum biologische Objekte, die ähnliches Verhalten zeigen, näher beieinander liegen als biochemisch entfernte Zellen. Durch die hierarchische Clusterung können biologische Objekte, die in ihrem Verhalten nahe beieinander liegen und somit ein Cluster bilden, von anderen Objekten, die weiter voneinander entfernt liegen, unterschieden werden. Die biologischen Objekte werden aufgrund ihrer Lage im Datenraum in natürliche Gruppen oder Cluster sortiert.

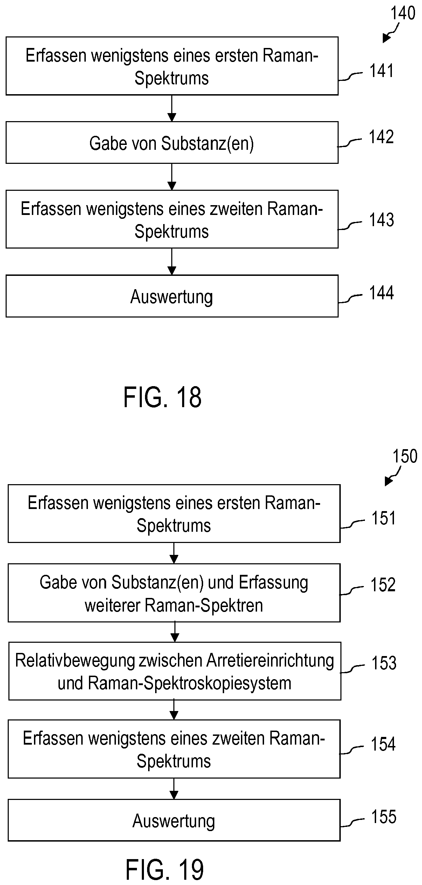

Figur 18 ist ein Flussdiagramm eines Verfahrens 140 nach einem Ausführungsbeispiel. Das Verfahren kann von der Vorrichtung 1 nach einem Ausführungsbeispiel automatisch ausgeführt werden.

Bei 141 wird wenigstens ein erstes Raman-Spektrum eines biologischen Objekts vor Gabe einer Substanz erfasst. Dabei kann das biologische Objekt von der Arretiereinrichtung arretiert sein.

Bei Schritt 142 wird dem biologischen Objekt eine Substanz zugeführt. Die Substanz kann ausgewählt sein aus einem Wirkstoff, einer Chemikalie, einem Toxin oder einer anderen Substanz.

Bei Schritt 143 wird während oder nach Gabe der Substanz wenigstens ein zweites Raman-Spektrum desselben biologischen Objekts erfasst. Der Aktor 22 der Vorrichtung 1 kann so gesteuert werden, dass dasselbe biologische Objekt wie bei Schritt 141 erneut angefahren wird, um während oder nach Gabe der Substanz das wenigstens eine zweite Raman-Spektrum zu erfassen.

Die Erfassung des ersten Raman-Spektrums bei Schritt 141 und/oder die Erfassung des wenigstens einen zweiten Raman-Spektrums bei Schritt 143 kann umfassen, dass das biologische Objekt durch eine Optische Pinzette oder ein anderes elektrisches oder magnetisches Wechselfeld zwischen einem Fluidstrom und einem Haltebereich der Arretiereinrichtung bewegt wird, um das biologische Objekt für die Messungen zu arretieren.

Die Erfassung des ersten Raman-Spektrums bei Schritt 141 und/oder die Erfassung des wenigstens einen zweiten Raman-Spektrums bei Schritt 143 kann eine Raman-Streuung am entsprechenden biologischen Objekt selbst beinhalten. Die Erfassung des ersten Raman-Spektrums bei Schritt 141 und/oder die Erfassung des wenigstens einen zweiten Raman-Spektrums bei Schritt 143 kann alternativ oder zusätzlich eine Raman-Streuung an einem Überstand, beispielsweise dem das biologische Objekt überdeckenden Fluid 82, oder einem anderen das biologische Objekt wenigstens teilweise umgebenden Material beinhalten.

Bei Schritt 144 kann eine Auswertung des ersten Raman-Spektrums und/oder wenigstens einen zweiten Raman-Spektrums des biologischen Objekts erfolgen. Durch die Auswertung kann die Reaktion des biologischen Objekts auf die Substanz ermittelt werden. Die Auswertung kann eine Hauptkomponentenanalyse, eine Clusteranalyse und/oder eine LDA umfassen, wie unter Bezugnahme auf Figur 1 bis Figur 17 beschrieben wurde. Die Auswertung kann optional die Unterdrückung von Signalanteilen, die durch die Arretiereinrichtung verursacht werden, umfassen. Dazu kann wenigstens ein Raman-Spektrum der Arretiereinrichtung in einem Bereich ohne biologisches Objekt erfasst werden, wie unter Bezugnahme auf Figur 19 näher beschrieben wird. Das wenigstens eine Raman-Spektrum der Arretiereinrichtung kann von dem bei Schritt 141 erfassten ersten Raman-Spektrum und/oder von dem bei Schritt 142 erfassten wenigstens einen zweiten Raman-Spektrum subtrahiert werden, um Signalanteile der Arretiereinrichtung von dem Signal des biologischen Objekts zu trennen.

Figur 19 ist ein Flussdiagramm eines Verfahrens 150 nach einem Ausführungsbeispiel. Das Verfahren kann von der Vorrichtung 1 nach einem Ausführungsbeispiel automatisch ausgeführt werden.

Bei Schritt 151 wird wenigstens ein erstes Raman-Spektrum eines biologischen Objekts erfasst, während das biologische Objekt von der Arretiereinrichtung arretiert ist. Das erste Raman-Spektrum kann vor, während oder nach Gabe einer Substanz, beispielsweise eines Wirkstoffes oder einer Chemikalie, erfasst werden.

Bei Schritt 152 kann eine Substanz, beispielsweise ein Wirkstoff oder einer Chemikalie, zugeführt werden. Dazu kann die Substanz über eine laminare oder nichtlaminare Strömung zu den biologischen Objekten transportiert werden. Anschließend können weitere Raman-Spektren erfasst werden.

Bei Schritten 151, 152 und optional den folgenden Schritten kann zunächst eine Zelle oder ein anderes biologisches Objekt durch Raman-Streuung gemessen, dann eine andere Zelle angefahren werden. Nach Gabe eines Wirkstoffes oder einer anderen Substanz und einer Wartezeit können alle bereits mit einer Raman-Spektroskopie untersuchten Zellen wieder einzeln angefahren und einer weiteren Raman-Spektroskopie unterzogen werden, nachdem die Substanz wenigstens eine vordefinierte Wartezeit lang auf die biologischen Objekte einwirken konnte.

Bei Schritt 153 kann der Aktor 22 gesteuert werden, um eine Relativbewegung zwischen der Arretiereinrichtung und dem Raman-Spektroskopiesystem hervorzurufen. Der Aktor 22 kann so gesteuert werden, dass bei der nachfolgend ausgeführten Erfassung an der Arretiereinrichtung kein biologisches Objekt durch das Raman-Spektroskopiesystem angeregt wird. Die Steuerung des Aktors 22 kann so erfolgen, dass die Arretiereinrichtung im Anregungsstrahl positioniert ist.

Bei Schritt 154 kann wenigstens ein zweites Raman-Spektrum erfasst werden, wobei kein biologisches Objekt im Anregungsstrahl des Raman-Spektroskopiesystems 10 positioniert ist. Das wenigstens eine zweite Raman-Spektrum kann die Raman-Streuung an der Arretiereinrichtung selbst repräsentieren.

Bei Schritt 155 kann eine Auswertung erfolgen, bei der die elektronische Recheneinrichtung 40 durch Bildung eines Differenzspektrums zwischen dem wenigstens einen ersten Raman-Spektrum, bei dem ein biologisches Objekt im Anregungsstrahl positioniert ist, und dem wenigstens einen zweiten Raman-Spektrum, bei dem kein biologisches Objekt im Anregungsstrahl positioniert ist, die von der Arretiereinrichtung stammenden Signalanteile im wenigstens einen ersten Raman-Spektrum unterdrückt. Es kann eine gewichtete Differenzbildung erfolgen, bei der das zweite Raman-Spektrum, das nur an der Arretiereinrichtung erfasst wurde, mit einem von eins verschiedenen Gewichtungsfaktor multipliziert wird, bevor es von dem ersten Raman-Spektrum subtrahiert wird. Der Gewichtungsfaktor kann aus einem Vergleich der Höhe eines für die Arretiereinrichtung charakteristischen Peaks in dem ersten Raman-Spektrum und in dem zweiten Raman-Spektrum ermittelt werden.

Die für Schritt 155 beschriebene Auswertung kann als Teil der Bestimmung der Reaktion biologischer Objekte auf eine Substanz bei Schritt 144 ausgeführt werden.

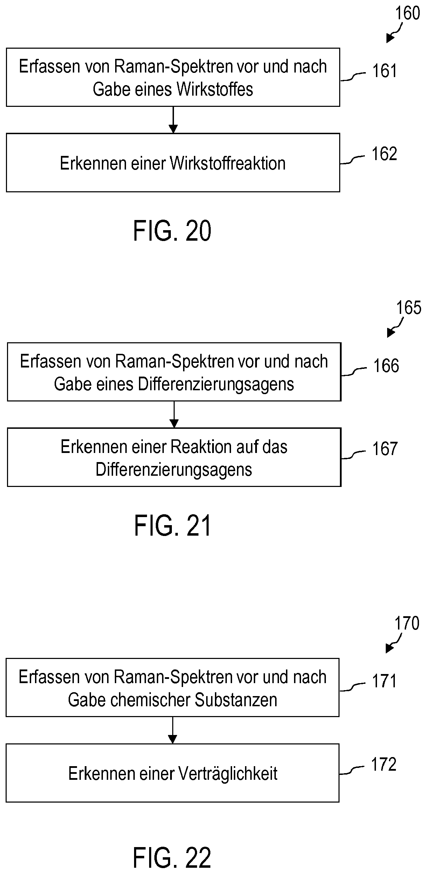

Die Verfahren und Vorrichtungen nach unterschiedlichen Ausführungsbeispielen können für verschiedene Zwecke eingesetzt werden, wie anhand von Figur 20 und Figur 22 beispielhaft veranschaulicht wird.

Figur 20 ist ein Flussdiagramm eines Verfahrens 160 nach einem Ausführungsbeispiel. Das Verfahren kann von der Vorrichtung 1 nach einem Ausführungsbeispiel automatisch ausgeführt werden. Das Verfahren kann zur Ermittlung von Wirkstoffresistenzen und/oder zur anderweitigen Ermittlung geeigneter Wirkstoffe eingesetzt werden.

Bei Schritt 161 werden Raman-Spektren eines biologischen Objekts oder mehrerer biologischer Objekte erfasst, bevor ein Wirkstoff zugegeben wird und während oder nachdem der Wirkstoff zugegeben wird. Der Wirkstoff kann ein Antibiotikum umfassen.

Bei Schritt 162 werden durch Auswertung der erfassten Raman-Spektren Reaktionen von Zellen auf den Wirkstoff erkannt. Dazu kann ermittelt werden, ob Keime oder Zellen als Reaktion auf den Wirkstoff in einen funktionell veränderten und/oder beeinträchtigten Zustand übergehen. Der Übergang in einen derartigen Zustand kann durch eine Hauptkomponentenanalyse, eine Clusteranalyse, eine LDA oder andere statistische Auswertungsmethoden erkannt werden.

Ein Beispiel der Reaktion der Zelle oder eines anderen biologischen Objekts auf einen Wirkstoff kann sein, dass mit dem Verfahren eine Wirkstoffresistenz des biologischen Objekts erkannt wird.

Alternativ oder zusätzlich kann bei Schritt 162 erkannt werden, ob beispielsweise nach Gabe von Differenzierungsstoffen sich die Zelle in einen funktionell veränderten Zustand verändert. Beispielsweise kann der Übergang von einem toti- oder multipotenten Zustand in einen funktionell veränderten Zustand erkannt werden.

Der Übergang in einen derartigen Zustand kann durch eine Hauptkomponentenanalyse, eine Clusteranalyse, eine LDA oder andere statistische Auswertungsmethoden erkannt werden.

Figur 21 ist ein Flussdiagramm eines Verfahrens 165 nach einem Ausführungsbeispiel. Das Verfahren kann von der Vorrichtung 1 nach einem Ausführungsbeispiel automatisch ausgeführt werden. Das Verfahren kann zur Ermittlung eine Reaktion auf ein Differenzierungsagens eingesetzt werden.

Bei Schritt 165 werden Raman-Spektren eines biologischen Objekts oder mehrerer biologischer Objekte erfasst, bevor ein Differenzierungsagens zugegeben wird und während oder nachdem das Differenzierungsagens zugegeben wird.

Bei Schritt 166 wird durch Auswertung der erfassten Raman-Spektren erkannt, ob nach Gabe von Differenzierungsstoffen sich eine Zelle in einen funktionell veränderten Zustand verändert. Beispielsweise kann der Übergang von einem toti- oder multipotenten Zustand in einen funktionell veränderten Zustand, beispielsweise einen ausdifferenzierten Zustand, erkannt werden. Der Übergang in einen derartigen Zustand kann durch eine Hauptkomponentenanalyse, eine Clusteranalyse, eine LDA oder andere statistische Auswertungsmethoden erkannt werden.

Figur 22 ist ein Flussdiagramm eines Verfahrens 170 nach einem Ausführungsbeispiel. Das Verfahren kann von der Vorrichtung 1 nach einem Ausführungsbeispiel automatisch ausgeführt werden. Das Verfahren kann zur Ermittlung der Verträglichkeit von Chemikalien oder anderen Stoffen eingesetzt werden.

Bei Schritt 171 werden Raman-Spektren eines biologischen Objekts oder mehrerer biologischer Objekte erfasst, bevor eine Chemikalie zugegeben wird und während oder nachdem die Chemikalie zugegeben wird.

Bei Schritt 172 wird durch Auswertung der erfassten Raman-Spektren eine Verträglichkeit der entsprechenden Chemikalie bestimmt. Dazu kann ermittelt werden, ob Zellen, Zellhaufen oder Zellklone als Reaktion auf die Chemikalie in einen funktionell beeinträchtigten Zustand, beispielsweise in Apoptose, übergehen. Der Übergang in einen derartigen Zustand kann durch eine Hauptkomponentenanalyse, eine Clusteranalyse, eine LDA oder andere statistische Auswertungsmethoden erkannt werden.

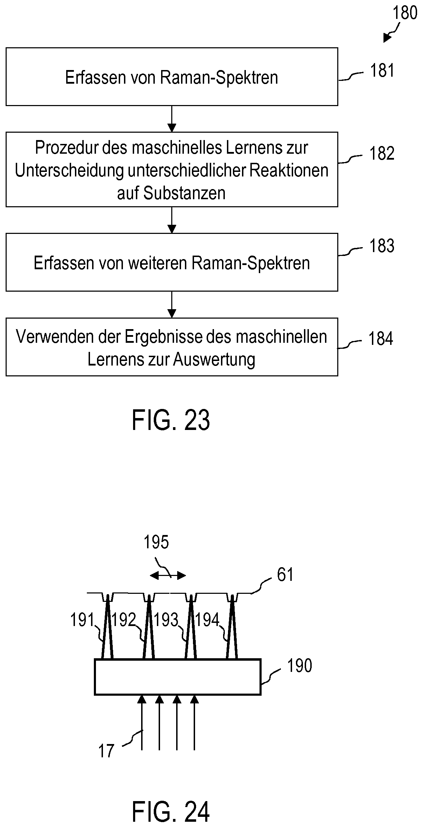

Figur 23 ist ein Flussdiagramm eines Verfahrens 180 nach einem Ausführungsbeispiel. Das Verfahren 180 kann von der Vorrichtung 1 ausgeführt werden. Das Verfahren 180 kann zur Ermittlung der Reaktion biologischer Objekte auf Substanzen verwendet werden. Regeln, nach denen die Reaktion biologischer Objekte auf Substanzen automatisch beurteilt wird, können von der Vorrichtung 1 mit einer Methode maschinellen Lernens, insbesondere durch beaufsichtigtes Lernen, automatisch erlernt werden. Die Regeln können nicht-flüchtig in dem Speicher der Recheneinrichtung 40 gespeichert werden.

Bei Schritt 181 werden mehrere Raman-Spektren erfasst. Die mehreren Raman-Spektren können gesunden und funktionell beeinträchtigten Zellen, Keimen oder anderen biologischen Objekten erfasst werden.

Bei Schritt 182 kann eine Prozedur maschinellen Lernens ausgeführt werden. Die Prozedur kann ein überwachtes Lernen sein. Die Vorrichtung 1 kann dabei für unterschiedliche erfasste Raman-Spektren eine Benutzereingabe erhalten. Die Benutzereingabe kann Raman-Spektren, Raman-Peaks und/oder Cluster einer Clusteranalyse unterschiedlichen Typen biologischer Objekte, beispielsweise unterschiedlichen Zelltypen oder unterschiedlichen Keimen, zuordnen. Die Benutzereingabe kann Raman-Spektren, Raman-Peaks und/oder Cluster einer Clusteranalyse unterschiedlichen Typen funktionellen Zuständen, beispielsweise einem lebenden Zustand und einem apoptotischen Zustand, zuordnen.

Anhand der Benutzereingabe kann die Vorrichtung 1 einen oder mehrere Parameter eines Regelsatzes einstellen, anhand derer die Vorrichtung 1 Raman-Spektren auswertet, um die Reaktion biologischer Objekte auf Substanzen zu bewerten. Anhand der Benutzereingabe kann die Recheneinrichtung 40 beispielsweise einen oder mehrere Parameter einer Stützvektormaschine anpassen, mit der erfasste Raman-Spektren ausgewertet werden, um die Reaktion biologischer Objekte auf Substanzen zu beurteilen.

Die erlernten Regeln, beispielsweise die Parameter der Stützvektormaschine, können von der Vorrichtung 1 in dem Speicher 43 gespeichert werden.

Bei Schritt 183 können Raman-Spektren an einem zu prüfenden biologischen Objekten erfasst werden, denen eine Substanz zugeführt wird.

Bei Schritt 184 können die gespeicherten Regeln auf die bei Schritt 183 erfassten Raman-Spektren angewendet werden. Dies kann beispielsweise wie unter Bezugnahme auf Figur 15 bis Figur 17 beschrieben erfolgen. Anhand der Regeln kann aus den erfassten Raman-Spektren ermittelt werden, welche Zelltypen oder Keime vorliegen. Anhand der Regeln kann aus den erfassten Raman-Spektren ermittelt werden, ob biologische Objekte funktionellen Veränderungen gegenüber voll funktionsfähigen biologischen Objekten unterliegen.

Auf diese Weise kann eine Mehrzahl von Klassen oder Clustern identifiziert werden. Durch den Vergleich der Spektren in jeder Klasse mit den Spektren bereits identifizierter Zellen, beispielsweise aus einer Reinkultur von Melanozyten oder anderen relevanten Zellen kann jeder Klasse ein Zelltyp zugeordnet werden. Die Anzahl der Zellen in jeder Klasse kann zur quantitativen Bestimmung der Mengenverhältnisse verwendet werden. Die Anzahl der Zellen in jeder Klasse geteilt durch die Gesamtzahl der gemessenen Spektren kann dann das Verhältnis des jeweiligen Zelltyps in dem Material quantitativ angeben, beispielweise in einem apoptotischen Zustand sind.

Die Vorrichtung und das Verfahren nach Ausführungsbeispielen können so ausgestaltet sein, dass in einer Multiplex-Technik die Raman-Spektren mehrerer biologischer Objekte parallel, insbesondere zeitgleich, erfasst werden können. Dazu kann ein Anregungsstrahl in eine Mehrzahl von Anregungsstrahlen aufgeteilt werden, wie anhand von Figur 24 bis Figur 27 näher beschrieben wird.

Figur 24 veranschaulicht die Erzeugung einer Mehrfach-Konfiguration zur simultanen Erfassung mehrerer Raman-Spektren. Ein optisches Element 190 ist eingerichtet, um einen Anregungsstrahl 17 des Raman-Spektroskopiesystems 10 in eine Mehrzahl von Anregungsstrahlen 191-194 umzusetzen. Das optische Element 190 kann einen räumlichen Lichtmodulator, ein oder mehrere diffraktive Elemente oder andere Einheiten umfassen. Das optische Element 190 kann elektronisch steuerbar sein, um die Anzahl und/oder Anordnung der Mehrzahl von Anregungsstrahlen 191-194 einzustellen. Die Vorrichtung 1 kann eingerichtet sein, um das optische Element 190 abhängig von der Ausgestaltung der Arretiereinrichtung 61 anzusteuern.

Jeder der Mehrzahl von Anregungsstrahlen 191-194 kann eine Optische Falle, z.B. wie oben beschrieben, erzeugen, in der ein biologisches Objekts zur Raman-Spektroskopie gehalten wird. Das biologische Objekt kann eine einzelne Zelle sein. Jeder der Mehrzahl von Anregungsstrahlen 191-194 kann somit sowohl zur Erzeugung einer optischen Falle als auch zur Anregung der Raman-Streuung dienen.

Das optische Element 190 kann so angesteuert werden, dass die mehreren optischen Fallen in unterschiedlichen Mikrowells der Arretiereinrichtung 61, die ein Mikrofluidikchip sein kann, positioniert ist. Die Mehrzahl von Anregungsstrahlen 191-194 kann so erzeugt werden, dass ihr Abstand abgestimmt ist auf einen Abstand 195 zwischen den Volumenmittelpunkten der Arretierbereiche, die beispielsweise Mikrowells eines Mikrofluidikchips sein können.

Eine Mehrfach-Konfiguration, bei der gleichzeitig jedes von mehreren biologischen Objekten mit einem ihm jeweils zugeordneten Anregungsstrahl angeregt wird, kann auch verwendet werden, wenn mehrere biologische Objekte in einer Vertiefung der Arretiereinrichtung 61, die ein Mikrofluidikchip sein kann, gehalten werden. Die mehreren optischen Fallen können dann eine reguläre oder irreguläre Anordnung von biologischen Objekten während der Raman-Spektroskopie definieren.

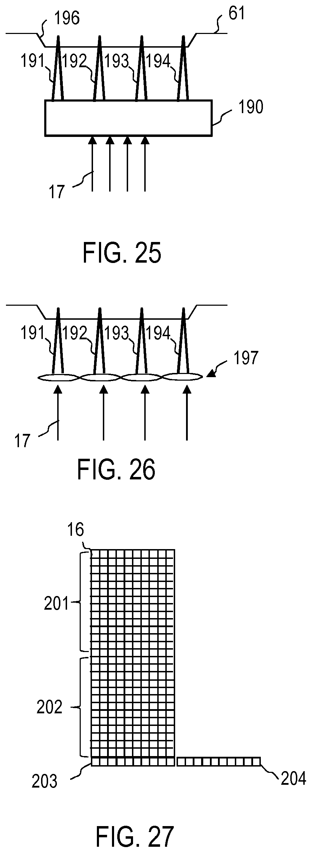

Figur 25 veranschaulicht die Erzeugung einer Mehrfach-Konfiguration zur simultanen Erfassung mehrerer Raman-Spektren. Das optische Element 190 ist eingerichtet, um einen Anregungsstrahl 17 des Raman-Spektroskopiesystems 10 in eine Mehrzahl von Anregungsstrahlen 191-194 umzusetzen. Das optische Element 190 kann einen räumlichen Lichtmodulator, ein oder mehrere diffraktive Elemente oder andere Einheiten umfassen. Das optische Element 190 kann elektronisch steuerbar sein, um die Anzahl und/oder Anordnung der Mehrzahl von Anregungsstrahlen 191-194 einzustellen.

Jeder der Mehrzahl von Anregungsstrahlen 191-194 erzeugt eine Optische Falle, in der ein biologisches Objekts zur Raman-Spektroskopie in einer Vertiefung 196 gehalten wird, wie oben beschrieben. Jeder der Mehrzahl von Anregungsstrahlen 191-194 hält ein ihm zugeordnetes biologisches Objekt in einer optischen Falle und dient zur Anregung der Raman-Streuung dienen.

Das optische Element 190 kann so angesteuert werden, dass die mehreren optischen Fallen in unterschiedlichen Abständen in der Vertiefung 196 des Mikrofluidikchips 2 positioniert sind. Die Mehrzahl von Anregungsstrahlen 191-194 kann so erzeugt werden, dass ihr Abstand abgestimmt ist auf eine durchschnittliche Größe der biologischen Objekte, die in der Vertiefung 196 gehalten werden.

Figur 26 zeigt eine Implementierung des optischen Elements 190, die bei Vorrichtungen nach Ausführungsbeispielen verwendet werden kann. Das optische Element 190, das im Strahlengang des Anregungsstrahls 17 positioniert wird, kann ein diffraktives Element sein oder kann ein diffraktives Element umfassen. Das optische Element 190 kann eine Anordnung 197 mehrerer diffraktiver Elemente umfassen. Das optische Element 190 kann ein Array 197 diffraktiver Elemente sein.

Alternativ oder zusätzlich kann das optische Element 190 eine elektrisch steuerbare Einrichtung umfassen. Die elektrisch steuerbare Einrichtung kann ein räumlicher Lichtmodulator oder eine andere Einrichtung sein, die steuerbar ist, um unterschiedliche Muster optischer Fallen zu erzeugen.

Eine Ausgestaltung, bei der ein Anregungsstrahl 17 in eine Mehrzahl von Anregungsstrahlen 191-194 aufgeteilt wird, erlaubt die simultane Anregung von Raman-Streuung in mehreren biologischen Objekten, die in den optischen Fallen der mehreren Anregungsstrahlen 191-194 gehalten werden. Die gestreuten Photonen können gleichzeitig durch einen Detektorchip derart erfasst werden, dass die einzelnen Spektren unterscheidbar bleiben. Beispielsweise kann die Erfassung des Raman-Streulichts derart erfolgen, dass die Signale unterschiedlicher biologischer Objekte so auf einen Kamerachip in unterschiedlichen Bildzeilen und/oder Bildspalten des Kamerachips erfasst und dann sequentiell ausgelesen werden.

Figur 27 zeigt einen Kamerachip des Detektors des Raman-Spektroskopiesystems, der zum Erfassen des Raman-Streulichts mehrerer biologischer Objekts verwendet werden kann. Der Kamerachip 16 kann ein EMCCD ("Electron multiplying chargecoupled device") sein.

Der Kamerachip 16 kann eingerichtet sein, um eine Multiplikation von Bildsignalen auszuführen, die auch als On-Chip-Multiplikation bezeichnet wird. Der Kamerachip 16 kann ein oder mehrere Register 181 zum Erfassen von Raman-Streulicht umfassen. Der Kamerachip 16 kann optional ein oder mehrere Register 182 zum Zwischenspeichern erfasster Spektren umfassen. Der Kamerachip 16 kann wenigstens ein Ausleseregister 183 umfassen.

Der Kamerachip 16 kann eingerichtet sein, um die Ladungen unterschiedlicher Sensorzeilen wenigstens zum Auslesen in benachbarte Sensorzeilen weiterzuschieben.

Der Kamerachip 16 kann ein Multiplikationsregister 184 umfassen. In dem Multiplikationsregister 184 kann eine Verstärkung der erfassten Ladungen im Ausleseregister 183 erfolgen. Dazu kann beispielsweise ein in der Technik als "Clock-Induced Charge" oder "Spurious Charge" bekannter Verstärkungsprozess genutzt werden.

Durch die Bilderfassung in Kombination mit Verschiebung der Ladungen zwischen Sensorzeilen und Multiplikation im Kamerachip 16 kann eine Filterung im Orts- oder Fourierraum erreicht werden, mit der die Raman-Streulichtsignale aus unterschiedlichen biologischen Fallen unterscheidbar sind.

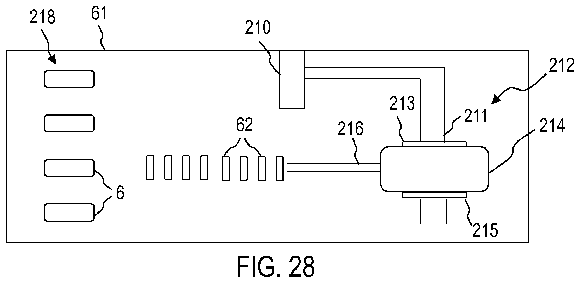

Figur 28 zeigt eine Draufsicht einer Ausgestaltung einer Arretiereinrichtung 61 einer Vorrichtung nach einem Ausführungsbeispiel. Die Arretiereinrichtung 61 kann als Mikrofluidikchip ausgestaltet sein.

Die Arretiereinrichtung 61 kann eine Einrichtung 212 zum Aufkonzentrieren von Bestandteilen der Probe aufweisen. Die Einrichtung 212 kann eingerichtet sein, um selektiv einen oder mehrere zelluläre oder andere Bestandteile der Probe aufzukonzentrieren. Dazu können osmotische Effekte eingesetzt werden. Es kann wenigstens eine Membran 213, 215 verwendet werden, die unterschiedliche Durchlässigkeiten für wenigstens zwei unterschiedliche Typen biologischer Objekte aufweist. Beispielsweise kann die Membran 213, 215 jeweils eine unterschiedliche Durchlässigkeit für zelluläre Blutbestandteile und für Verunreinigungen, beispielsweise Bakterien, Pilze, Hefe, Viren oder andere Verunreinigungen, aufweisen.

Wenigstens ein Teilvolumen einer Probe kann über einen Eingangskanal 210 und einen Verbindungskanal 211 der Einrichtung 212 zum Aufkonzentrieren zugeführt werden.

Die Einrichtung 212 zum Aufkonzentrieren umfasst wenigstens ein Element 213, dessen Durchlässigkeit für bestimmte Zellen, beispielsweise Blutzellen, von einer Durchlässigkeit für Keime, Bakterien oder anderen Verunreinigungen verschieden ist. Das Element 213 kann beispielsweise so ausgestaltet sein, dass es zelluläre Blutbestandteile eine höhere Durchlässigkeit aufweist als für Bakterien. Das Element 213 kann eine Membran sein. Das Element 213 kann von dem Teilvolumen der Probe durchströmt werden, so dass aufgrund der selektiven Durchlässigkeit native Zellen oder Verunreinigungen in einem Bereich 214 der Arretiereinrichtung 61 aufkonzentriert werden.

Die Arretiereinrichtung 61 kann auch wenigstens eine weitere Membran 215 zur Aufkonzentration umfassen. Die wenigstens eine weitere Membran 215 kann so ausgestaltet sein, dass ihre Durchlässigkeit für bestimmte Zellen, beispielsweise Blutzellen, von einer Durchlässigkeit für Keime, Bakterien oder anderen Verunreinigungen verschieden ist.

Aus dem Bereich 214, in dem biologische Objekte eines Typs oder mehrerer unterschiedlicher Typen aufkonzentriert werden, können die aufkonzentrierten biologischen Objekte über wenigstens einen Verbindungskanal 216 in einem Fluidstrom einen oder mehreren Aufnahmebereichen 62 zugeführt werden. In den einen oder mehreren Aufnahmebereichen 62 können sich die biologischen Objekte durch Wirkung der Schwerkraft absetzen oder unter Verwendung optischer oder anderer elektromagnetischer Strahlungsfelder in die Vertiefungen gezogen werden. In den Aufnahmebereichen 62 können die biologischen Objekte einer Raman-Spektroskopie unterzogen werden. Dabei kann jeweils mindestens ein Raman-Spektrum vor Gabe einer Substanz und wenigstens ein weiteres Raman-Spektrum während oder nach Gabe der Substanz erfasst werden.

Die biologischen Objekte können zu einer Mehrzahl 218 von Sammelaufnahmebereichen 6 geführt werden. Dazu können biologische Objekte abhängig von einem Ergebnis der Raman-Spektroskopie selektiv in einen Fluidstrom überführt werden, der die Aufnahmebereiche 62 und wenigstens einen der Sammelaufnahmebereiche 6 überströmt. Das Überführen kann unter Verwendung einer optischen Pinzette oder durch andere optische oder elektromagnetische Strahlungsfelder erfolgen.

Der Fluidstrom zwischen den Aufnahmebereichen 62 und den Sammelaufnahmebereichen 6 kann zeitabhängig veränderlich sein. Beispielsweise kann die Richtung des Fluidstroms derart kontrolliert werden, dass zeitsequentiell unterschiedliche der Sammelaufnahmebereiche 6 von dem Fluidstrom überströmt werden. Abhängig davon, in welchen der Sammelaufnahmebereiche 6 ein biologisches Objekt aufgrund seiner Raman-Spektren vor und nach Gabe einer Substanz überführt werden soll, kann der Zeitpunkt bestimmt werden, zu dem biologische Objekte aus den Aufnahmebereichen 62 in den Fluidstrom überführt werden, der sie zu dem gewünschten Sammelaufnahmebereich 6 transportiert.

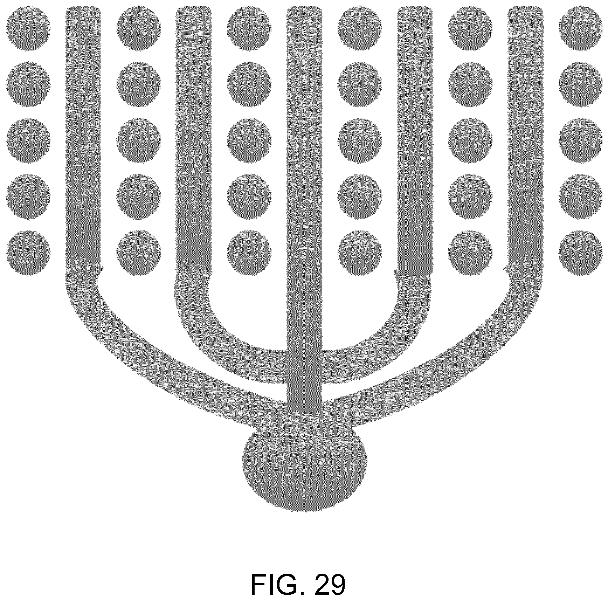

Figur 29 zeigt eine Draufsicht einer Ausgestaltung einer Arretiereinrichtung einer Vorrichtung nach einem Ausführungsbeispiel. Die Arretiereinrichtung kann Aufnahmebereiche umfassen, welche als Microwells zum Aufnehmen biologischer Objekte ausgestaltet sind. Die Aufnahmebereiche können entlang von Kanälen angeordnet sein, welche von Fluidströmen durchströmt sind. Dazu können biologische Objekte abhängig zum Beispiel von einem Ergebnis der Raman-Spektroskopie selektiv in einen Fluidstrom überführt werden. Das Überführen kann unter Verwendung einer optischen Pinzette oder durch andere optische oder elektromagnetische Strahlungsfelder erfolgen.

Die Anordnung der Kanäle kann einem hierarchischen Muster folgen mit einem Hauptast und einem oder mehreren Nebenästen, welche an verschiedenen Positionen vom Hauptast abzweigen können. Die Anordnung beinhaltet weiter einen Sammelaufnahmebereich, in welchen alle Äste direkt oder indirekt über den Hauptast einmünden. Zusätzlich können an allen Positionen weitere Subverästelungen vorliegen. Durch die Anordnung der Aufnahmebereiche in der Arretiereinrichtung ist die automatisierte Untersuchung mehrerer biologischer Objekte möglich. Die Anzahl der gleichzeitig untersuchbaren Objekte kann zwischen 1 bis 1000 liegen. Beispielsweise können 10, 20, 30, 40, 50, 100 oder mehr biologische Objekte gleichzeitig oder auch zeitversetzt untersucht werden. Darüber hinaus ist die Zufuhr von Substanzen in alle Bereiche möglich. Die Substanzen können entweder identisch sein, oder es können unterschiedliche Substanzen in die Aufnahmebereiche verbracht werden. Weiterhin können unterschiedliche Sektoren der Aufnahmebereiche mit unterschiedlichen Substanzen versehen werden.

Die verästelte Anordnung der Aufnahmebereiche in der Arretiereinrichtung erlaubt es darüber hinaus, bestimmte biologische Objekte gem. Untersuchungsergebnissen oder jedweder anderen Logik aus den Aufnahmebereichen in die Kanäle zu überführen und so zu sortieren. Eine Gruppe von biologischen Objekten, welche z.B. ähnliche Reaktionen auf Substanzen wie oben beschrieben gezeigt haben, kann so in einen Sammelaufnahmebereich transportiert werden, um sie dort zu sammeln, ggf. weiter zu separieren, weiteren Untersuchungen zuzuführen oder sie einer Kultivierung oder Expansion zuzuführen. Alternativ kann die Anordnung auch zur Entfernung von biologischen Objekten verwendet werden, welche nicht weiter benötigt werden, z. B. weil sie Untersuchungsparameter nicht erfüllt haben. Diese biologischen Objekte können zunächst in einen Sammelaufnahmebereich verbracht und anschließend entsorgt werden.
Die Arretiereinrichtung, welche Microwells zum Aufnehmen biologischer Objekte in verästelnder Ausgestaltung mit zu- bzw. abführenden Kanälen aufweist, erlaubt es somit, biologische Objekte nach Bedarf in einen der Kanäle zu verschieben, dort abtransportieren und zu sortieren.

Alternativ kann ein biologisches Objekt aus dem Sammelaufnahmebereich mittels Fluidstrom, welcher kontrollierbar ist, in Richtung spezifischer Aufnahmebereiche oder Aufnahmen transportiert werden. Dort kann es mittels Fluidstrom eingespült oder unter Verwendung einer Optischen Pinzette oder durch andere optische oder elektromagnetische Strahlungsfelder in die Aufnahme d.h. ein Microwell, eingetragen werden. Innerhalb des Microwell kann sich ein biologisches Objekt dann beispielsweise unter Wirkung der Schwerkraft absetzen. Ein erneutes Heraustransportieren ist gem. den oben genannten Ausführungsformen ebenfalls möglich.

Die Vorrichtungen und Verfahren nach Ausführungsbeispielen wie hierin beschrieben können zur Überprüfung einer Vielzahl unterschiedlicher biologischer Objekte und/oder einer Vielzahl unterschiedlicher Substanzen eingesetzt werden. Beispielsweise können die Vorrichtungen und Verfahren nach Ausführungsbeispielen verwendet werden, um geeignete Wirkstoffe automatisch oder computerassistiert zu identifizieren, um personalisierte Therapien automatisch oder computerassistiert zu entwickeln, oder um Verträglichkeiten von Substanzen, beispielsweise zur Verwendung in Kosmetika, automatisch zu beurteilen.

Vorrichtungen und Verfahren nach Ausführungsbeispielen können allgemein zur quantitativen Untersuchung von biologischen Objekten eingesetzt werden, die von einer Arretiereinrichtung wenigstens vorübergehend arretiert werden.

## Patentansprüche

1. Vorrichtung zum Analysieren biologischer Objekte (3, 4; 52; 53), umfassend
ein Raman-Spektroskopiesystem (10) zum Erfassen wenigstens eines Raman-Spektrums (101, 102),
eine Arretiereinrichtung (5; 51; 61; 91; 95), die eingerichtet ist, um die biologischen Objekte (3, 4; 52; 53) wenigstens vorübergehend zu arretieren, und
eine elektronische Recheneinrichtung (40), die eingerichtet ist, um abhängig von einer Auswertung des wenigstens einen Raman-Spektrums (101, 102) eine Reaktion eines von der Arretiereinrichtung (5; 51; 61; 91; 95) arretierten biologischen Objekts (3, 4; 52; 53) auf wenigstens eine Substanz (31, 32) zu ermitteln,
wobei die Vorrichtung so ausgestaltet ist, dass die biologischen Objekte so angeordnet werden, dass sie zeitlich getrennt und einzeln erfasst werden können,
wobei die Vorrichtung kein SERS-Raman-Spektroskopiesystem ist, **dadurch gekennzeichnet, dass** die Arretiereinrichtung (5; 51; 61; 91; 95) Haltebereiche (62; 63; 92) umfasst, an denen jeweils mindestens ein biologisches Objekt (3, 4; 52; 53) arretierbar ist, wobei die Vorrichtung (1) eingerichtet ist, um einen über die Mehrzahl von Haltebereichen (62; 63; 92) strömenden Fluidstrom (82) zu erzeugen,
wobei die Arretierung in einer Vertiefung oder einem Microwell erfolgt;
weiterhin umfassend eine Zuführeinrichtung (30) zum Zuführen der wenigstens einen Substanz (31, 32) zu den biologischen Objekten (3, 4; 52; 53).

2. Vorrichtung nach Anspruch 1,
wobei die Vorrichtung eingerichtet ist, um das von der Arretiereinrichtung (5; 51; 61; 91; 95) arretierte biologische Objekt (3, 4; 52; 53) wiederholt zum Erfassen des wenigstens einen Raman-Spektrums (101, 102) anzufahren, vorzugsweise umfassend einen von der elektronischen Recheneinrichtung (40) steuerbaren Aktor (22) zum Hervorrufen einer Relativbewegung zwischen der Arretiereinrichtung (5; 51; 61; 91; 95) und dem Raman-Spektroskopiesystem (10).

3. Vorrichtung nach Anspruch 1 oder Anspruch 2, umfassend
eine mit der elektronischen Recheneinrichtung (40) gekoppelte Bilderfassungseinrichtung (19), die eingerichtet ist, um ein Bild der durch die Arretiereinrichtung (5; 51; 61; 91; 95) arretierten biologischen Objekte (3, 4; 52; 53) zu erfassen,
wobei die elektronische Recheneinrichtung (40) eingerichtet ist, um den Aktor (22) abhängig von dem erfassten Bild zu steuern, um Mikrowells nach der Gabe der Substanz und zu bestimmten Zeiten automatisch anzufahren und Raman-Spektren zu messen.

4. Vorrichtung nach einem der Ansprüche 1 bis 3,
wobei die Haltebereiche (62) jeweils so dimensioniert sind, dass an jedem Haltebereich (62) nur genau ein biologisches Objekt (3, 4; 52; 53) arretierbar ist,
wobei die Haltebereiche (62) vorzugsweise jeweils so dimensioniert sind, dass an jedem Haltebereich (62) nur genau eine Zelle arretierbar ist.

5. Vorrichtung nach einem der vorhergehenden Ansprüche,
wobei die elektronische Recheneinrichtung (40) eingerichtet ist, um abhängig von der Auswertung des wenigstens einen Raman-Spektrums (101, 102) die Reaktion auf eine Substanz oder mehrere Substanzen (31, 32) zu ermitteln und/oder um abhängig von der Auswertung mehrerer Raman-Spektren (101, 102) einen Verlauf der Reaktion zeitabhängig zu verfolgen.

6. Vorrichtung nach einem der vorhergehenden Ansprüche,
wobei die elektronische Recheneinrichtung (40) eingerichtet ist, um abhängig von der Auswertung des wenigstens einen Raman-Spektrums (101, 102) eine Wirkstoffresistenz zu erkennen,
wobei die elektronische Recheneinrichtung (40) vorzugsweise eingerichtet ist, um zum Erkennen der Wirkstoffresistenz ein vor Zuführen der wenigstens einen Substanz (31, 32) erfasstes erstes Raman-Spektrum (101) mit einem nach einer Wirkstoffgabe erfassten zweiten Raman-Spektrum (102) zu vergleichen.

7. Vorrichtung nach einem der vorhergehenden Ansprüche,
wobei die wenigstens eine Substanz (31, 32) einen Wirkstoff, ein Toxin und/oder eine Chemikalie umfasst.

8. Vorrichtung nach einem der vorhergehenden Ansprüche,
wobei das wenigstens eine von der elektronischen Recheneinrichtung (40) ausgewertete Raman-Spektrum (101, 102) durch Raman-Streuung an einem biologischen Objekt (3, 4; 52; 53) oder an einem von dem biologischen Objekt (3, 4; 52; 53) verschiedenen Material (81) erfasst wird.

9. Vorrichtung nach einem der vorhergehenden Ansprüche,
wobei die Vorrichtung (1) eingerichtet ist, um parallel die Reaktion mehrerer biologischer Objekte (3, 4; 52; 53) auf eine Substanz (31, 32) oder mehrere Substanzen (31, 32) zu ermitteln,
wobei die Vorrichtung (1) eingerichtet ist, um einen Anregungsstrahl des Raman-Spektroskopiesystems aufzuteilen, um mehrere elektromagnetische Strahlen zum Halten mehrerer biologischer Objekte (3, 4; 52; 53) und zur Raman-Spektroskopie zu erzeugen.

10. Vorrichtung nach einem der vorhergehenden Ansprüche,
wobei die Vorrichtung (1) eingerichtet ist, um abhängig von einer Auswertung des wenigstens einen Raman-Spektrums (101, 102) eines von der Arretiereinrichtung (5; 51; 61; 91; 95) arretierten biologischen Objekts (3, 4; 52; 53), dieses selektiv zu entnehmen und/oder zu sortieren.

11. Verfahren zum Analysieren biologischer Objekte (3, 4; 52; 53), umfassend:
Arretieren eines biologischen Objekts (3, 4; 52; 53) an Haltebereiche (62; 63; 93),
Erfassen wenigstens eines Raman-Spektrums (101, 102) des arretierten biologischen Objekts (3, 4; 52; 53), und
Auswerten des wenigstens einen Raman-Spektrums (101, 102) zum Ermitteln einer Reaktion eines biologischen Objekts (3, 4; 52; 53) auf wenigstens eine Substanz (31, 32), wobei die biologischen Objekte so angeordnet werden, dass sie zeitlich getrennt und einzeln erfasst werden können, und
wobei das Verfahren nicht auf einem SERS-Raman-Spektroskopiesystem durchgeführt wird;
wobei die biologischen Objekte (3, 4; 52; 53) über die Mehrzahl von Haltebereichen (62; 63; 92) in einem Fluidstrom transportiert werden und die Arretierung in einer Vertiefung oder einem Microwell erfolgt;
und wobei die wenigstens eine Substanz (31, 32) zu den biologischen Objekten (3, 4; 52; 53) mittels einer Zuführeinrichtung (30) zugeführt wird,
wobei vorzugsweise die wenigstens eine Substanz (31, 32) einen Wirkstoff, ein Toxin und/oder eine Chemikalie umfasst, wobei das Verfahren optional von der Vorrichtung (1) gemäß einem der Ansprüche 1 bis 10 automatisch ausgeführt wird.

12. Verfahren nach Anspruch 11,
wobei nach Auswerten des wenigstens einen Raman-Spektrums (101, 102) zum Ermitteln einer Reaktion eines biologischen Objekts (3, 4; 52; 53) auf wenigstens eine Substanz (31, 32), das biologische Objekt selektiv entnommen und/oder sortiert wird.

## Claims

1. A device for analyzing biological objects (3, 4; 52; 53), comprising
a Raman spectroscopy system (10) for acquiring at least one Raman spectrum (101, 102),
an arresting unit (5; 51; 61; 91; 95) that is configured to at least temporarily arrest the biological objects (3, 4; 52; 53), and
an electronic computing unit (40) that is configured to determine a reaction of a biological object (3, 4; 52; 53) arrested by the arresting unit (5; 51; 61; 91; 95) to at least one substance (31, 32) in accordance with an evaluation of the at least one Raman spectrum (101, 102)
wherein the device is designed in such a way that the biological objects are arranged so that they can be detected separately in time and individually,
wherein the device is not a SERS Raman spectroscopy system,
**characterized in that** the arresting device (5; 51; 61; 91; 95) comprises holding areas (62; 63; 92) at each of which at least one biological object (3, 4; 52; 53) is arrestable,
wherein the device (1) is configured to produce a fluid stream (82) flowing over the plurality of holding areas (62; 63; 92),
wherein the arresting takes place in a recess or a microwell;
further comprising a supply unit (30) for supplying the at least one substance (31, 32) to the biological objects (3, 4; 52; 53).

2. The device as claimed in claim 1,
wherein the device is configured to repeatedly engage the biological object (3, 4; 52; 53) arrested by the arresting unit (5; 51; 61; 91; 95) in order to acquire the at least one Raman spectrum (101, 102),
preferably comprising an actuator (22) controllable by the electronic computing unit (40) for producing a relative movement between the arresting unit (5; 51; 61; 91; 95) and the Raman spectroscopy system (10).

3. The device as claimed in claim 1 or claim 2, comprising
an image acquisition device (19) coupled to the electronic computing unit (40) that is configured to acquire an image of the biological objects (3, 4; 52; 53) arrested by the arresting unit (5; 51; 61; 91; 95),
wherein the electronic computing unit (40) is configured to control the actuator (22) in accordance with the acquired image in dependence on the acquired image to automatically engage microwells after administration of the active substance and at specified times and to measure Raman spectra.

4. The device as claimed in one of claims 1 to 3,
wherein each of the holding areas (62) is dimensioned such that only exactly one biological object (3, 4: 52; 53) is arrestable at each holding area (62)
wherein each of the holding areas (62) is preferably dimensioned such that only exactly one cell is arrestable at each holding area (62).

5. The device as claimed in one of the preceding claims,
wherein the electronic computing unit (40) is configured, in accordance with the evaluation of the at least one Raman spectrum (101, 102), to determine the reaction to a substance or a plurality of substances (31, 32) and/or, in accordance with the evaluation of a plurality of Raman spectra (101, 102), to follow a course of the reaction in a time-dependent manner.

6. The device as claimed in one of the preceding claims,
wherein the electronic computing unit (40) is configured to detect a drug resistance in accordance with the evaluation of the at least one Raman spectrum (101, 102)
wherein the electronic computing unit (40) is preferably configured, in order to detect the drug resistance, to compare a first Raman spectrum (101) acquired before the supply of the at least one substance (31, 32) with a second Raman spectrum acquired after administration of an active substance (102).

7. The device as claimed in any of the preceding claims, wherein the at least one substance (31, 32) comprises an active substance, a toxin and/or a chemical.

8. The device as claimed in one of the preceding claims,
wherein the at least one Raman spectrum (101, 102) evaluated by the electronic computing unit (40) is acquired by Raman scattering on a biological object (3, 4; 52; 53) or a material (81) different from the biological object (3, 4; 52; 53).

9. The device as claimed in any of the preceding claims,
wherein the device (1) is configured to determine in parallel the reaction of a plurality of biological objects (3, 4; 52; 53) to a substance (31, 32) or a plurality of substances (31, 32),
wherein the device (1) is configured to split an excitation beam of the Raman spectroscopy system in order to produce a plurality of electromagnetic beams for holding a plurality of biological objects (3, 4; 52; 53) and for Raman spectroscopy.

10. The device as claimed in one of the preceding claims,
wherein the device (1) is configured, in accordance with an evaluation of the at least one Raman spectrum (101, 102) of a biological object (3, 4; 52; 53) arrested by the arresting unit (5; 51; 61; 91; 95), to selectively remove and/or sort said biological object.

11. A method for analyzing biological objects (3, 4; 52; 53), comprising:
arresting of a biological object (3, 4; 52; 53) in holding areas (62; 63; 92),
acquisition of at least one Raman spectrum (101, 102) of the arrested biological object (3, 4; 52; 53), and
evaluation of the at least one Raman spectrum (101, 102) in order to determine a reaction of a biological object (3, 4; 52; 53) to at least one substance (31, 32)
wherein the biological objects are arranged so that they can be detected separately in time and individually
wherein the method is not performed on a SERS Raman spectroscopy system
wherein the biological objects (3, 4; 52; 53) are transported in a fluid stream over the plurality of holding areas (62; 63; 92) and the arresting takes place in a recess or a microwell;
and wherein the at least one substance (31, 32) is supplied to the biological objects (3, 4; 52; 53) by means of a supply unit (30),
wherein preferably the at least one substance (31, 32) comprises an active substance, a toxin and/or a chemical, wherein the method is optionally performed automatically by the device (1) as claimed in one of claims 1 to 10.

12. The method as claimed in claim 11,
wherein after evaluation of the at least one Raman spectrum (101, 102) in order to determine a reaction of a biological object (3, 4; 52; 53) to at least one substance (31, 32), the biological object is selectively removed and/or sorted.

## Revendications

1. Dispositif d'analyse d'objets biologiques (3, 4 ; 52 ; 53), comprenant
un système de spectroscopie Raman (10) pour détecter au moins un spectre Raman (101, 102),
un dispositif de blocage (5 ; 51 ; 61 ; 91 ; 95), qui est adapté pour bloquer au moins temporairement les objets biologiques (3, 4 ; 52 ; 53), et
un dispositif de calcul électronique (40), qui est adapté pour déterminer une réaction d'un objet biologique (3, 4 ; 52 ; 53) bloqué par le dispositif de blocage (5 ; 51 ; 61 ; 91 ; 95) sur au moins une substance (31, 32), en fonction d'une évaluation du au moins un spectre Raman (101, 102),
dans lequel le dispositif est conçu de telle sorte que les objets biologiques sont agencés de manière à pouvoir être séparés dans le temps et détectés individuellement,
dans lequel le dispositif n'est pas un système de spectroscopie SERS-Raman,
**caractérisé en ce que**,
le dispositif de blocage (5 ; 51 ; 61 ; 91 ; 95) comprend des zones de retenue (62 ; 63 ; 92), au niveau desquelles au moins un objet biologique (3, 4 ; 52 ; 53) peut être bloqué, dans lequel le dispositif (1) est adapté pour générer un courant de fluide (82) s'écoulant via la pluralité de zones de retenue (62 ; 63 ; 92),
dans lequel le blocage a lieu dans une cavité ou un micropuits ;
comprenant en outre un dispositif d'alimentation (30) pour alimenter la au moins une substance (31, 32) vers les objets biologiques (3, 4 ; 52 ; 53).

2. Dispositif selon la revendication 1,
dans lequel le dispositif est adapté pour démarrer de façon répétée l'objet biologique (3, 4 ; 52 ; 53) bloqué par le dispositif de blocage (5 ; 51 ; 61 ; 91 ; 95) en vue de détecter au moins un spectre Raman (101, 102), comprenant de préférence un actionneur (22) pouvant être commandé par le dispositif de calcul électronique (40) en vue de provoquer un mouvement relatif entre le dispositif de blocage (5 ; 51 ; 61 ; 91 ; 95) et le système de spectroscopie Raman (10).

3. Dispositif selon la revendication 1 ou la revendication 2, comprenant
un dispositif de capture d'image (19) couplé au dispositif de calcul électronique (40), qui est conçu pour capturer une image des objets biologiques (3, 4 ; 52 ; 53) bloqués par le dispositif de blocage (5 ; 51 ; 61 ; 91 ; 95),
dans lequel le dispositif de calcul électronique (40) est adapté pour commander l'actionneur (22) en fonction de l'image capturée, pour démarrer automatiquement les micropuits après l'administration de la substance et à des moments définis et pour mesurer des spectres Raman.

4. Dispositif selon l'une quelconque des revendications 1 à 3,
dans lequel les zones de retenue (62) sont chacune dimensionnées de telle sorte qu'un seul objet biologique (3, 4 ; 52 ; 53) peut être bloqué au niveau de chaque zone de retenue (62),
dans lequel les zones de retenue (62) sont de préférence chacune dimensionnées de telle sorte qu'une seule cellule exactement peut être bloquée au niveau de chaque zone de retenue (62).

5. Dispositif selon l'une quelconque des revendications précédentes,
dans lequel le dispositif de calcul électronique (40) est adapté pour déterminer la réaction à une substance ou plusieurs substances (31, 32) en fonction de l'évaluation d'au moins un spectre Raman (101, 102) et/ou pour suivre temporellement, en fonction de l'évaluation de plusieurs Spectres Raman (101, 102), une évolution de la réaction.

6. Dispositif selon l'une quelconque des revendications précédentes,
dans lequel le dispositif de calcul électronique (40) est adapté pour identifier une résistance du principe actif en fonction de l'évaluation du au moins un spectre Raman (101, 102),
dans lequel le dispositif de calcul électronique (40) est de préférence conçu, à des fins d'identification de la résistance du principe actif, pour comparer un premier spectre Raman (101) détecté préalablement à l'apport de la au moins une substance (31, 32) avec un second spectre Raman (102) détecté après une administration de principe actif.

7. Dispositif selon l'une quelconque des revendications précédentes,
dans lequel la au moins une substance (31, 32) comprend un principe actif, une toxine et/ou un produit chimique.

8. Dispositif selon l'une quelconque des revendications précédentes,
dans lequel le au moins un spectre Raman (101, 102) analysé par le dispositif de calcul électronique (40) est détecté par diffusion Raman sur un objet biologique (3, 4 ; 52 ; 53) ou sur un matériau (81) différent de l'objet biologique (3, 4 ; 52 ; 53).

9. Dispositif selon l'une quelconque des revendications précédentes,
dans lequel le dispositif (1) est adapté pour déterminer en parallèle la réaction de plusieurs objets biologiques (3, 4 ; 52 ; 53) sur une substance (31, 32) ou sur plusieurs substances (31, 32),
dans lequel le dispositif (1) est adapté pour diviser un faisceau d'excitation du système de spectroscopie Raman afin de générer plusieurs faisceaux électromagnétiques en vue de retenir plusieurs objets biologiques (3, 4 ; 52 ; 53) et en vue d'une spectroscopie Raman.

10. Dispositif selon l'une quelconque des revendications précédentes,
dans lequel le dispositif (1) est adapté pour prélever et/ou trier sélectivement un objet biologique (3, 4 ; 52 ; 53) bloqué par le dispositif de blocage (5 ; 51 ; 61 ; 91 ; 95) en fonction d'une évaluation du au moins un spectre Raman (101, 102) de cet objet biologique.

11. Procédé d'analyse d'objets biologiques (3, 4 ; 52 ; 53), comprenant les étapes consistant à :
bloquer un objet biologique (3, 4 ; 52 ; 53) au niveau de zones de retenue (62 ; 63 ; 93),
détecter au moins un spectre Raman (101, 102) de l'objet biologique bloqué (3, 4 ; 52 ; 53), et
analyser le au moins un spectre Raman (101, 102) pour déterminer une réaction d'un objet biologique (3, 4 ; 52 ; 53) sur au moins une substance (31, 32), les objets biologiques étant agencés de manière à pouvoir être séparés dans le temps et détectés individuellement, et
dans lequel le procédé n'est pas réalisé sur un système de spectroscopie SERS-Raman ;
dans lequel les objets biologiques (3, 4 ; 52 ; 53) sont transportés via la pluralité de zones de retenue (62 ; 63 ; 92) dans un courant de fluide et le blocage survient dans une cavité ou un micropuits ;
et dans lequel la au moins une substance (31, 32) est alimentée vers les objets biologiques (3, 4 ; 52 ; 53) au moyen d'un dispositif d'alimentation (30),
dans lequel de préférence la au moins une substance (31, 32) comprend un principe actif, une toxine et/ou un produit chimique, dans lequel le procédé est exécuté automatiquement en option par le dispositif (1) selon l'une quelconque des revendications 1 à 10.

12. Procédé selon la revendication 11,
dans lequel, après l'analyse du au moins un spectre Raman (101, 102) en vue de déterminer une réaction d'un objet biologique (3, 4 ; 52 ; 53) sur au moins une substance (31, 32), l'objet biologique est prélevé et/ou trié de manière sélective.
